(19) Europäisches Patentamt
European Patent Office
Office européen des brevets

(11) **EP 3 799 064 A1**

(12) **EUROPEAN PATENT APPLICATION**

(43) Date of publication:
**31.03.2021 Bulletin 2021/13**

(51) Int Cl.:
**G16H 40/20** (2018.01)  **G06Q 50/00** (2012.01)
**G16H 10/60** (2018.01)

(21) Application number: **20180447.3**

(22) Date of filing: **17.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.09.2019 JP 2019176451**

(71) Applicant: **Hitachi, Ltd.**
**Tokyo 100-8280 (JP)**

(72) Inventors:
• **YUI, Shuntaro**
**Chiyoda-ku, Tokyo 100-8280 (JP)**
• **BAN, Hideyuki**
**Chiyoda-ku, Tokyo 100-8280 (JP)**
• **OOSAKI, Takanobu**
**Chiyoda-ku, Tokyo 100-8280 (JP)**

(74) Representative: **Strehl Schübel-Hopf & Partner**
**Maximilianstrasse 54**
**80538 München (DE)**

(54) **ANALYSIS SYSTEM AND ANALYSIS METHOD**

(57) Provided is an analysis system that presents an effective diagnosis and treatment act for each patient. The analysis system includes an evaluation index calculation unit that calculates an index value indicating medical care quality based on received medical care act data and clinical data, a cost calculation unit that calculates, by using medical cost data, costs of a medical care act that relate to a disease name to be analyzed and are generated after a period of a detected disease developing event, and an extraction unit that extracts, by using at least one of the calculated index value and the calculated costs, the received medical care act and clinical data of a patient to be analyzed and an extracted correlation, a medical care act to be recommended to the patient to be analyzed.

FIG. 2

**Description**

BACKGROUND OF THE INVENTION

1. Field of the Invention

The present invention relates to an analysis system that analyzes healthcare data in the medical field.

2. Description of the Related Art

Due to a rapid change in a population structure such as declining birthrate and aging population, it is an urgent task to construct a sustainable medical care providing system in response to a rapid increase in medical costs. Given the variety of stakeholders, evidence-based decision-making is required to formulate a new system that guarantees medical care quality and optimizes medical costs. It is a global activity to use stored data, and data analysis is considered as an effective method for generating evidence. A high-quality medical care providing system can be constructed by providing incentives and penalties according to effects and effectiveness of diagnosis and treatment acts or measures that are extracted by analyzing data. In a current effectiveness analysis, the analysis is executed depending on whether a diagnosis and treatment act is executed or depending on a providing amount of the diagnosis and treatment act.

The background arts in the present technical field include the following related arts. JP-A-2014-215761 (Patent Literature 1) discloses a method for analyzing a situational change of a market. According to the method, time series of situation vectors including numeric values each for characterizing a rate of increase or decrease in market occupancy scale of each competition element at each point of time are calculated, a time series model of the situation vectors sequentially from a competition element in an uppermost level toward lower levels are created by referring to a hierarchical order relation in market competition of service category hierarchical information storage units when detecting a change in situation vectors for a given time section, and a change point in the situation vectors is detected as a situational change.

WO2015/77582 (Patent Literature 2) discloses a composition and a method for treating and improving symptoms of rheumatoid arthritis by using an antibody that specifically binds to a human interleukin-6 receptor (hIL-6R).

Another example of the related art includes B. Lee et al., Identification of Type 2 Diabetes Risk Factors Using Phenotypes Consisting of Anthropometry and Triglycerides Based on Machine Learning, IEEE J. of Biomedical and Health Informatics, vol. 20, No. 1, Jan. 2016 (Non-patent Literature 1). Non-patent Literature 1 discloses a technique for extracting risk factors of diabetes by using naive Bayes and binary logistic regression.

It is required to accurately evaluate an economic value of a diagnosis and treatment act or measure considered to be highly effective when determining an incentive method for the diagnosis and treatment act. However, since a database indicated by a medical bill includes much data loss called real world data, it is difficult to extract a cost effective diagnosis and treatment act or measure even if by using data described in the medical bill only. For example, a disease developing period may not be accurately known due to data loss, and various complications occurring after the disease develops may not be known.

Although Non-patent Literature 1 discloses a method for extracting risk factors of diabetes based on examination values, whether there is a diagnosis and treatment act or measure and time series information of the treatment act or measure are not considered. Although Patent Literature 1 discloses a system that detects a change point of a KPI and extracts a causal relationship, a temporal change of the causal relationship is not considered. Although an economic evaluation specialized for a component called Sarilumab is calculated in Patent Literature 2, an economic evaluation is not considered for a medicine whose component is not specialized, or a diagnosis and treatment act. As described above, from the viewpoint of an overall economic loss due to disease developing, it is difficult to effectively and efficiently present an economic evaluation of a highly effective diagnosis and treatment act or measure.

SUMMARY OF THE INVENTION

Therefore, the invention provides a technique for effectively and efficiently presenting an effective diagnosis and treatment act for each patient by using a medical care database including data loss.

A representative example of the invention disclosed in the present application is as follows. That is, an analysis system that analyzes an effect of a medical care act is implemented by a computer including an arithmetic device executing a predetermined processing and a storage device connected to the arithmetic device. The analysis system includes an input unit that receives medical care act data, clinical data including an examination result of a patient, medical cost data, and a medical care act and clinical data of a patient to be analyzed, an event detection unit that detects a disease developing event, a relationship extraction unit that extracts a correlation between the detected disease developing event and a medical care act based on time series information, an evaluation index calculation unit that calculates an index value indicating medical care quality based on the received medical care act data and clinical data,

**EP 3 799 064 A1**

a cost calculation unit that calculates, by using the medical cost data, costs of a medical care act that relate to a disease name to be analyzed and are generated after a period of the detected disease developing event, and an extraction unit that extracts, by using at least one of the calculated index value and the calculated costs, the received medical care act and clinical data of the patient to be analyzed and the correlation extracted by the relationship extraction unit, a medical care act to be recommended to the patient to be analyzed.

According to an aspect of the invention, an effective diagnosis and treatment act can be recommended for each patient. Problems, configurations, and effects other than those described above will become apparent from the following description of embodiments.

BRIEF DESCRIPTION OF THE DRAWINGS

FIG. 1 is a diagram showing a business model implemented by a diagnosis and treatment act and measure economic value evaluation system according to an embodiment of the invention.
FIG. 2 is a configuration diagram showing the diagnosis and treatment act and measure economic value evaluation system according to the present embodiment.
FIG. 3 is a hardware configuration diagram showing the diagnosis and treatment act and measure economic value evaluation system according to the present embodiment.
FIG. 4 is a flowchart schematically showing processings executed by the diagnosis and treatment act and measure economic value evaluation system according to the present embodiment.
FIG. 5 is a sequence diagram showing a part of the processings (S302 to S305) shown in FIG. 4.
FIG. 6 is a diagram showing an example of a condition setting and processing result display screen.
FIG. 7 is a flowchart showing step S302 in detail.
FIG. 8 is a diagram showing an example of diagnosis and treatment act data.
FIG. 9 is a diagram showing an example of clinical data.
FIG. 10 is a diagram showing an example of disease onset knowledge data.
FIG. 11 is a diagram showing an example of a disease onset date management table.
FIG. 12 is a flowchart showing step S303 in detail.
FIG. 13 is a sequence diagram showing a part of processings (S3031 to S3032) shown in FIG. 12.
FIG. 14 is a diagram showing a processing of generating a diagnosis and treatment act time series information table.
FIG. 15 is a diagram showing an example of a treatment act characteristic amount table.
FIG. 16 is a flowchart showing step S306 in detail.
FIG. 17 is a flowchart showing step S307 in detail.
FIG. 18 is a diagram showing an example of a related disease name table.
FIG. 19 is a diagram showing an example of a medical cost table.
FIG. 20 is a flowchart showing step S308 in detail.
FIG. 21 is a diagram showing an example of a condition setting and processing result display screen.
FIG. 22 is a diagram showing an example of a condition setting and processing result display screen.
FIG. 23 is a flowchart showing step S309 in detail.
FIG. 24 is a diagram showing an example of a condition setting and processing result display screen.
FIG. 25 is a diagram showing an example of a condition setting and processing result display screen.
FIG. 26 is a flowchart showing step S308 in detail according to a first modification.
FIG. 27 is a diagram showing an example of a condition setting and processing result display screen according to the first modification.
FIG. 28 is a diagram showing another example of the condition setting and processing result display screen according to the first modification.
FIG. 29 is a flowchart showing step S308 in detail according to the first modification.
FIG. 30 is a diagram showing an example of a condition setting and processing result display screen according to a second modification.
FIG. 31 is a diagram showing an example of a condition setting and processing result display screen according to the second modification.
FIG. 32 is a configuration diagram showing a diagnosis and treatment act and measure economic value evaluation system according to a third modification.
FIG. 33 is a flowchart showing step S309 in detail according to the third modification.
FIG. 34 is a diagram showing an example of a condition setting and processing result display screen according to the third modification.

DESCRIPTION OF THE PREFERRED EMBODIMENTS

Hereinafter, embodiments of the invention will be described with reference to the drawings.

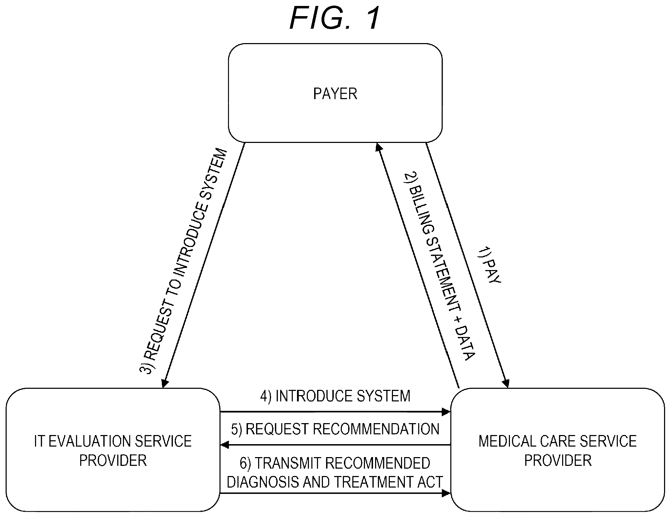

Before describing a diagnosis and treatment act and measure economic value evaluation system according to embodiments of the invention, an example of a business model implemented by the diagnosis and treatment act and measure economic value evaluation system will be described. FIG. 1 is a diagram showing the business model implemented by the diagnosis and treatment act and measure economic value evaluation system according to an embodiment of the invention. Main constituents in the business model shown in FIG. 1 includes a medical care service provider (such as a medical institution), a user (a payer), and an IT evaluation service provider.

The medical care service provider provides a medical care service (such as data of a diagnosis result) to the user (the payer). The user pays for the provided medical care service (1) and the medical care service provider sends a billing statement (2).

The IT evaluation service provider receives, from the user, a request to introduce the diagnosis and treatment act and measure economic value evaluation system (3), and introduces the system (4). When the medical care service provider (for example, a doctor) requests the diagnosis and treatment act and measure economic value evaluation system to recommend a medical care act (5), the IT evaluation service provider (the diagnosis and treatment act and measure economic value evaluation system) transmits the recommended diagnosis and treatment act to the medical care service provider (6). The IT evaluation service provider is also positioned as a BPO provider since the IT evaluation service provider presents the recommended diagnosis and treatment act by using the billing statement and necessary data (such as details of the medical care act) that are collected from the medical care service provider.

In this manner, even when there is information loss in one database such as data loss, according to the business model using the diagnosis and treatment act and measure economic value evaluation system according to the present embodiment, it is possible to accurately extract a cost effective diagnosis and treatment act for a disease based on an analysis result of previous medical care data, and to recommend a highly effective medical care act to the medical care service provider (for example, a doctor). In Japan, in particular, medical bills are collected nationwide, but so-called outcome information is missing. On the other hand, hospitals have the outcome information, but the number of patients is small. Therefore, it is difficult to predict a diagnosis and treatment act event that is most likely to occur subsequently.

From the viewpoint of preventing a disease from becoming severe, developing progresses of most diseases are known by accurately knowing a disease developing period of an underlying disease. However, the present system can recommend a cost effective medical care act even if the disease developing period is not accurately known. When calculating cost effectiveness of a diagnosis and treatment act, information of a plurality of separated databases is complemented without accessing detailed information, and cost effectiveness of a target disease can be accurately extracted. The business model shown in FIG. 1 is an example. The payer may not be presented, that is, the business model may be one in which a relationship between only the medical care service provider and the IT evaluation service provider is constructed and the economic evaluation system is introduced.

<First Embodiment>

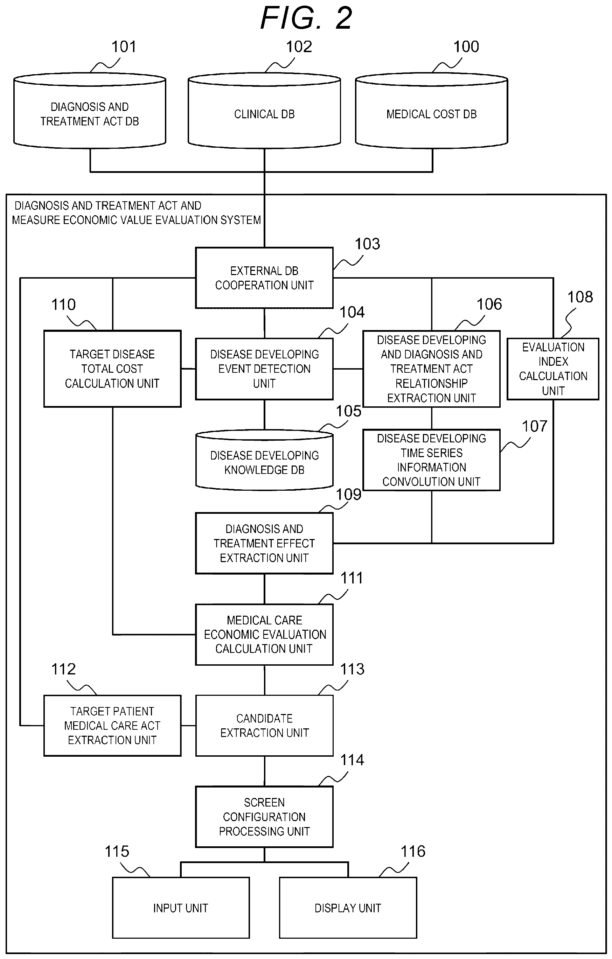

FIG. 2 is a configuration diagram showing the diagnosis and treatment act and measure economic value evaluation system according to the present embodiment.

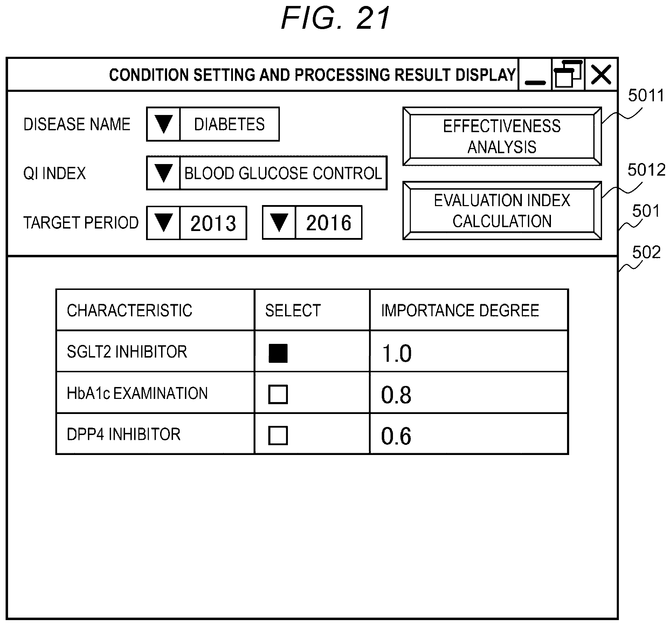

The system includes an external DB cooperation unit 103, a disease developing event detection unit 104, a disease developing knowledge database 105, a disease developing and diagnosis and treatment act relationship extraction unit 106, a disease developing time series information convolution unit 107, an evaluation index calculation unit 108, a diagnosis and treatment effect extraction unit 109, a target disease total cost calculation unit 110, a medical care economic evaluation calculation unit 111, a target patient medical care act extraction unit 112, a candidate extraction unit 113, a screen configuration processing unit 114, an input unit 115, and a display unit 116. The external DB cooperation unit 103 has a function of cooperating with a database outside the system. For example, the external DB cooperation unit 103 may acquire data stored in a diagnosis and treatment act database 101, a clinical database 102, and a medical cost database 100, and may cooperate with other databases. In the diagnosis and treatment act and measure economic value evaluation system according to the present embodiment, the disease developing and diagnosis and treatment act relationship extraction unit 106, the disease developing time series information convolution unit 107, the evaluation index calculation unit 108, and the diagnosis and treatment effect extraction unit 109 are not essential configurations, but are configurations necessary to display a selected index (an importance degree) of a highly effective diagnosis and treatment act or measure on condition setting and processing result display screens (shown in FIGS. 21, 27, 28, and 30), and any one or all may be provided.

The input unit 115 is an interface that receives an input from a user. The display unit 116 is an interface that outputs an execution result of a program in a format that can be visually confirmed by the user.

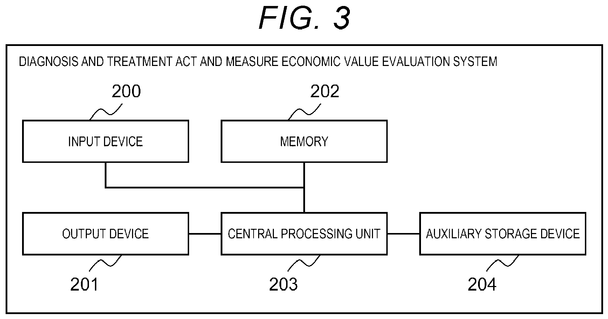

A hardware configuration of the system will be described. FIG. 3 is a hardware configuration diagram showing the diagnosis and treatment act and measure economic value evaluation system according to the present embodiment.

An input device 200 is a keyboard, a mouse, a pen tablet, or the like that forms the input unit 115. The input device 200 is an interface that receives an input from the user. An output device 201 is a display device such as a liquid crystal display device or a cathode-ray tube (CRT) that forms the display unit 116. The output device 201 is an interface that outputs an execution result of a program in a format that can be visually confirmed by the user. Alternatively, the output device 201 may be a device that outputs the execution result onto a paper medium such as a printer. The input device 200 and the output device 201 may be provided in a terminal connected to the diagnosis and treatment act and measure economic value evaluation system via a network.

A central processing unit 203 is a processor (an arithmetic device) that executes a program. Specifically, the processor executes programs to implement the external DB cooperation unit 103, the disease developing event detection unit 104, the disease developing and diagnosis and treatment act relationship extraction unit 106, the disease developing time series information convolution unit 107, the evaluation index calculation unit 108, the diagnosis and treatment effect extraction unit 109, the target disease total cost calculation unit 110, the medical care economic evaluation calculation unit 111, the target patient medical care act extraction unit 112, the candidate extraction unit 113, and the screen configuration processing unit 114. A part of processings performed by the processor executing the programs may be performed by another type (for example, by hardware) of an arithmetic device (such as a field programmable gate array (FPGA) and an application specific integrated circuit (ASIC)).

A memory 202 includes a ROM which is a non-volatile storage element and a RAM which is a volatile storage element. The ROM stores an invariable program (for example, a BIOS), or the like. The RAM is a high-speed and volatile storage element such as a dynamic random access memory (DRAM), and temporarily stores a program to be executed by a processor and data to be used when the program is executed.

An auxiliary storage device 204 is a large-capacity and non-volatile storage device such as a magnetic storage device (for example, an HDD) and a flash memory (for example, an SSD) . The auxiliary storage device 204 stores data to be used when the central processing unit 203 executes a program and the program to be executed by the central processing unit 203. Specifically, the auxiliary storage device 204 stores the disease developing knowledge database 105. A part of or the entire of the disease developing knowledge database 105 is stored in the memory 202 in a short time when the program is executed. The program is read from the auxiliary storage device 204, loaded into a memory, and executed by the central processing unit 203.

Although not shown, the diagnosis and treatment act and measure economic value evaluation system includes a communication interface that controls communication between the diagnosis and treatment act and measure economic value evaluation system and other devices in accordance with a predetermined protocol.

The program to be executed by the central processing unit 203 is introduced into the diagnosis and treatment act and measure economic value evaluation system via a removable medium (such as a CD-ROM and flash memory) or a network, and is stored in the non-volatile auxiliary storage device 204 which is a non-transitory storage medium. Therefore, the diagnosis and treatment act and measure economic value evaluation system may include an interface that reads data from the removable medium.

The diagnosis and treatment act and measure economic value evaluation system is a computer system formed by one physical computer or by a plurality of logical or physical computers, or may be operated on a virtual computer constructed on a plurality of physical computer resources.

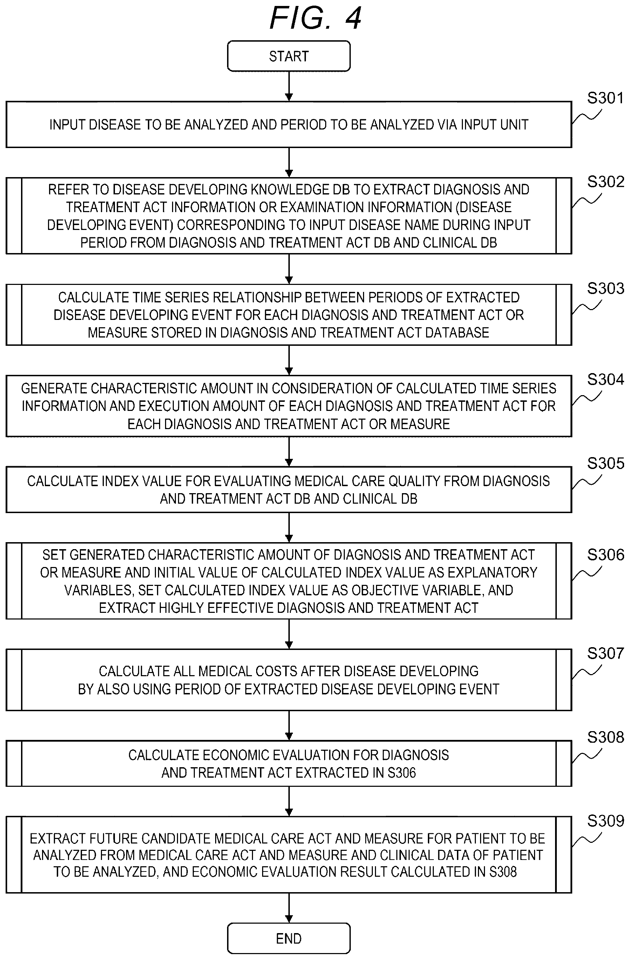

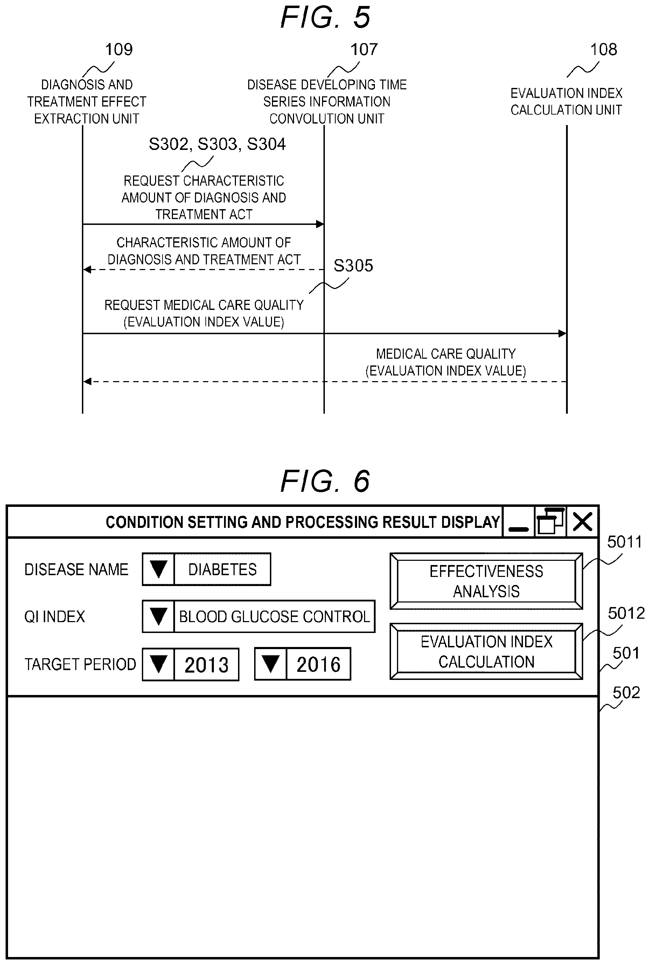

FIG. 4 is a flowchart schematically showing processings executed by the system according to the present embodiment. FIG. 5 is a sequence diagram showing a part of the processings (S302 to S305) shown in FIG. 4.

First, when the display unit 116 displays a condition setting and processing result display screen (shown in FIG. 6), the user inputs a disease (a disease name) to be analyzed, a QI index, and a period to be analyzed via the input unit 115 (S301).

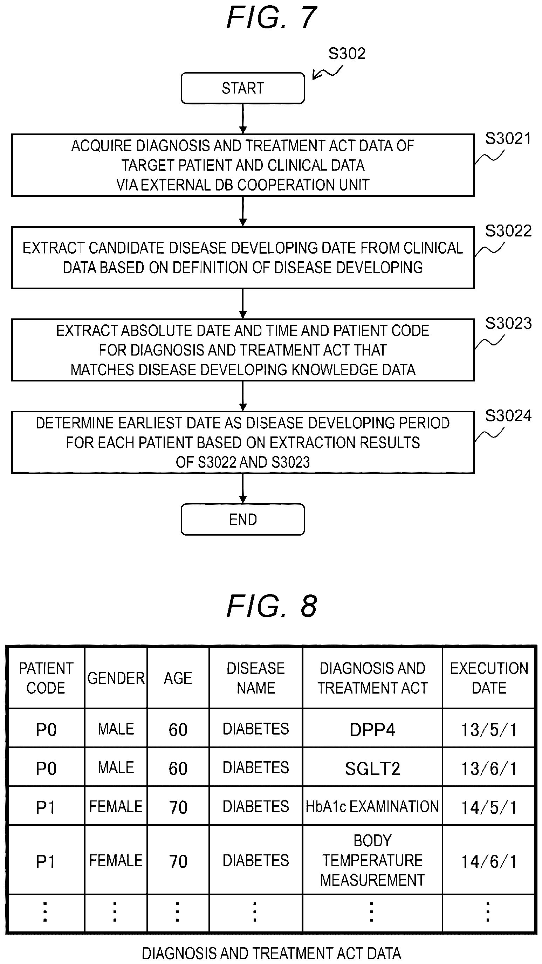

Next, the disease developing event detection unit 104 refers to the disease developing knowledge database 105 to extract diagnosis and treatment act information and examination information corresponding to the input disease name during the to-be-analyzed period input in step S301 from the diagnosis and treatment act database 101 and the clinical database 102 (S302). That is, information (a disease developing event) of a patient who may have developed the disease (the disease name) is extracted in step S302. The processing in step S302 will be described in detail with reference to FIG. 7.

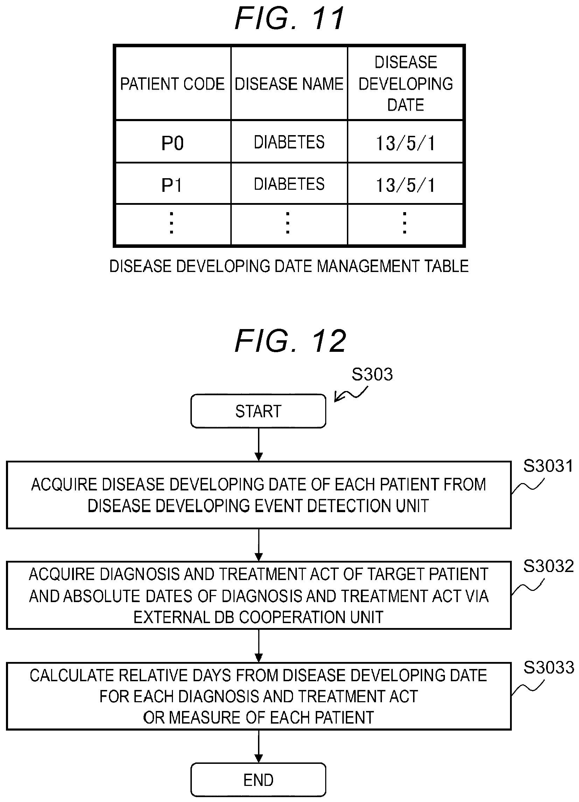

Then, the disease developing and diagnosis and treatment act relationship extraction unit 106 calculates a time series relationship (for example, relative days indicating a temporal relationship) between periods of the disease developing event extracted in S302 for each diagnosis and treatment act or measure stored in the diagnosis and treatment act database 101 (S303). The processing in step S303 will be described in detail in FIG. 12.

Next, the disease developing time series information convolution unit 107 generates a characteristic amount in consideration of the time series information calculated in S303 and an execution amount of each diagnosis and treatment act or measure for the diagnosis and treatment act or measure processed in S303 (S304).

Then, the evaluation index calculation unit 108 calculates an index value for evaluating medical care quality based on the diagnosis and treatment act database 101 and the clinical database 102 (S305).

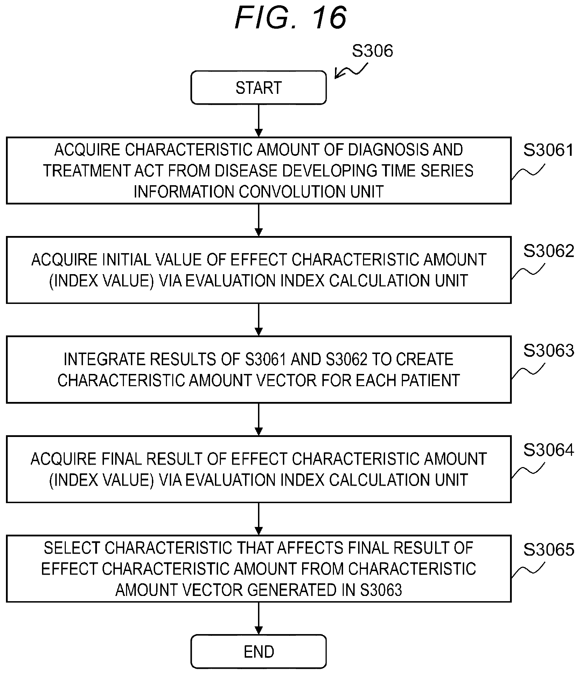

Next, the diagnosis and treatment effect extraction unit 109 sets the characteristic amount of the diagnosis and treatment act or measure generated in S304 and an initial value of a characteristic amount of the index value calculated in S305 as explanatory variables, sets an effect characteristic amount (for example, an index value) calculated in S305 as an objective variable, and extracts a highly effective diagnosis and treatment act or measure (S306) . The processing in step S306 will be described in detail with reference to FIG. 16. The initial value of the characteristic amount of the index value is used to recognize a change amount of the index value during the period to be analyzed, and can fill a difference between basic values among patients . If there is no difference between the basic values among patients, it is not necessary to use the initial value.

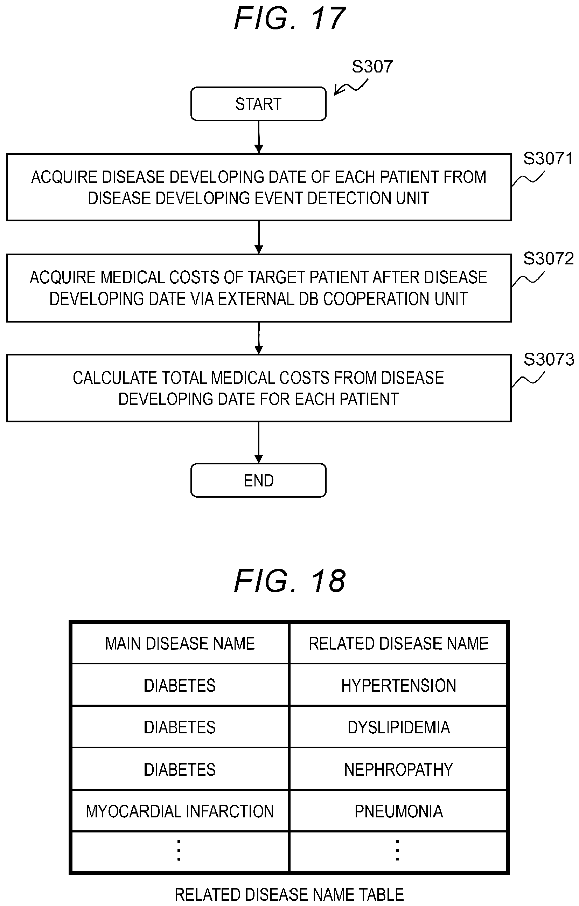

Then, the target disease total cost calculation unit 110 calculates, using the period of the extracted disease developing event, all medical costs after the disease develops (S307). The processing in step S307 will be described in detail with reference to FIG. 17.

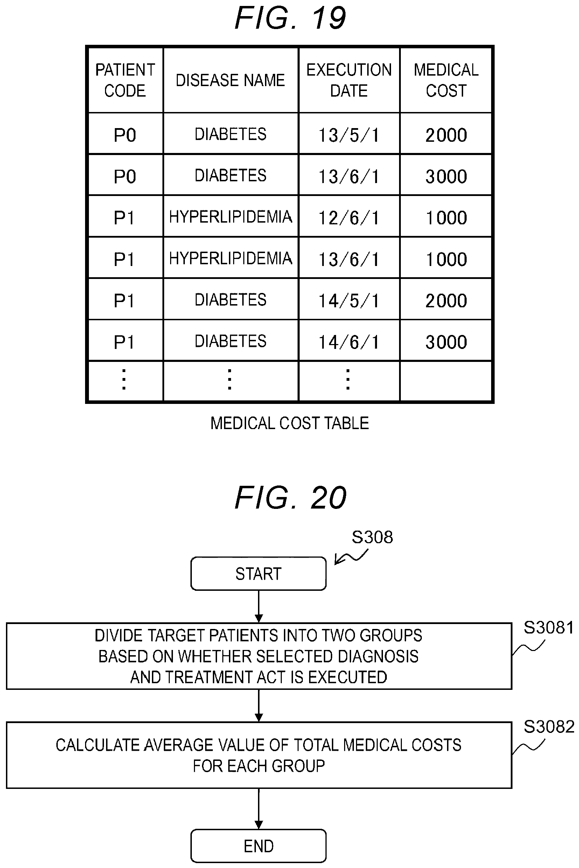

Next, the medical care economic evaluation calculation unit 111 calculates an economic evaluation for the diagnosis and treatment act extracted in S306 (S308). The processing in step S308 will be described in detail in FIG. 20.

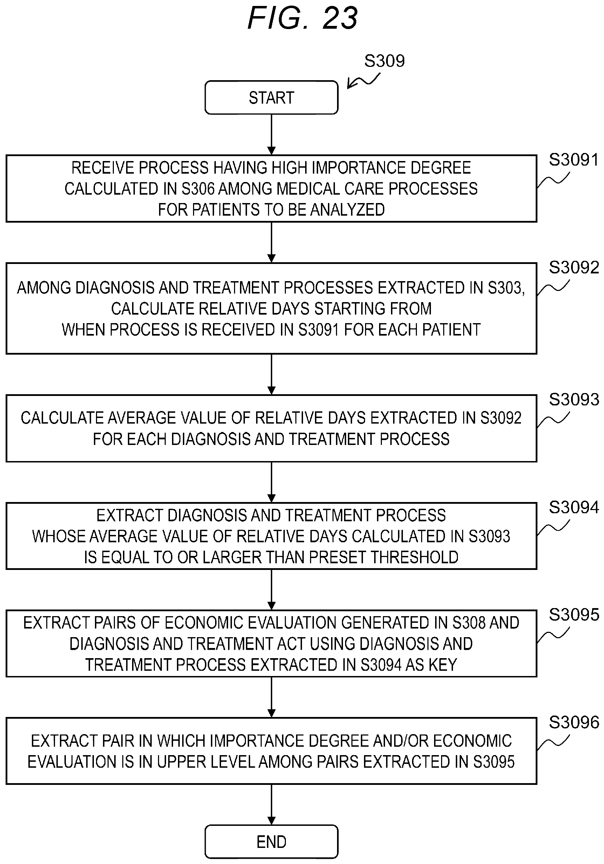

Finally, a future candidate medical care act or measure of a patient to be analyzed is extracted from the medical care act or measure and clinical data of the patient to be analyzed and the economic evaluation result calculated in S308 (S309). The processing in step S309 is executed by the candidate extraction unit 113 and the screen configuration processing unit 114. The processing in S309 will be described in detail with reference to FIG. 23.

FIG. 6 is a diagram showing an example of the condition setting and processing result display screen displayed by the display unit 116 in step S301.

The condition setting and processing result display screen includes a condition setting region 501 and a processing result presentation region 502. The condition setting region 501 displays an "effectiveness analysis" button 5011 that is operated to analyze effectiveness of a diagnosis and treatment act, an "evaluation index calculation" button 5012 that is operated to evaluate an economic value of a diagnosis and treatment act or measure, and a pull-down selection input field for setting an analysis condition. In the example shown in FIG. 6, a condition is set for extracting a diagnosis and treatment act or measure having a high effect of affecting blood glucose control by using data of a diabetes patient from year 2013 to year 2016, and nothing is displayed in the processing result presentation region 502.

Next, step S302 will be described in detail with reference to FIG. 7.

First, the disease developing event detection unit 104 acquires diagnosis and treatment act or measure data of a target patient from the diagnosis and treatment act database 101 and acquires clinical data from the clinical database 102 via the external DB cooperation unit 103 (S3021).

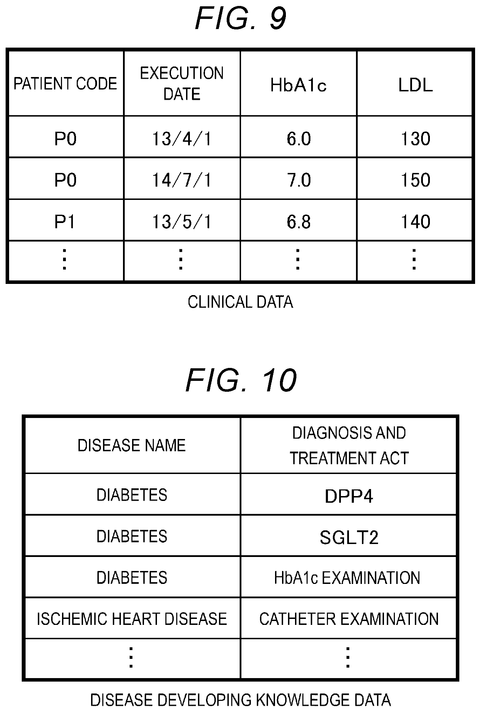

As shown in FIG. 8, the diagnosis and treatment act data stored in the diagnosis and treatment act database 101 includes a patient attribute (such as a patient code, gender, and age), a diagnosis and treatment act (such as a prescribed medicine and an examination content), and an execution date. In addition to the diagnosis and treatment act executed by a medical care institution, the diagnosis and treatment act data may include measures other than the diagnosis and treatment act (for example, health guidance, diet guidance, and regular exercise). As shown in FIG. 9, the clinical data stored in the clinical database 102 includes a patient code, an examination execution date, and an examination value. The diagnosis and treatment act database 101 is not limited to one in which all diagnosis and treatment acts or measures executed on a patient are correctly input, and may be one including data loss. An examination recorded in the clinical database 102 may not be regularly received by a patient. That is, an input loss may occur in either one of the diagnosis and treatment act database 101 and the clinical database 102.

Next, the disease developing event detection unit 104 extracts a candidate disease developing date from the clinical data based on a definition of the disease developing (S3022) . In the case of diabetes, diabetes develops when a value of HbAlc is equal to or larger than 6.5% of a threshold as defined in clinical guidelines. July, 2014 is extracted as a candidate disease developing date for a patient having a patient code P0 and May, 2013 is extracted as a candidate disease developing date for a patient having a patient code P1.

Then, the disease developing event detection unit 104 extracts absolute dates (an execution date) and a patient code for a diagnosis and treatment act or measure that matches disease developing knowledge data extracted from the disease developing knowledge database 105 (S3023). As shown in FIG. 10, the disease developing knowledge data records a relationship between a disease and a diagnosis and treatment act or measure. Specifically, a DPP 4 inhibitor prescription, an SGLT2 inhibitor prescription, and an HbAlc examination are recorded as diagnosis and treatment acts related to diabetes. (P0, DPP4, 13/5/1), (P0, SGLT2, 13/6/1), (P1, HbAlc examination, 14/5/1) can be extracted by using data shown in FIGS. 8 to 10. Since body temperature measurement is not defined in the disease developing knowledge data, the body temperature measurement is not extracted in step S3023.

Although the disease developing knowledge database 105 is registered in advance in the present embodiment, the diagnosis and treatment act or measure related to a disease may be extracted using the diagnosis and treatment act data and the clinical data. For example, a disease developing knowledge generation unit may be provided. The disease developing knowledge generation unit may refer to the diagnosis and treatment act data and the clinical data to extract a diagnosis and treatment act or measure related to a disease and construct the disease developing knowledge database 105. Specifically, a diagnosis and treatment act or measure having a high correlation with a disease name in the diagnosis and treatment act data is extracted, or a diagnosis and treatment act or measure having a high correlation with a disease developing definition (for example, a value of the HbAlc is 6.5% or more) in the clinical data is extracted.

Next, an earliest date and time is determined as a disease developing period for each patient based on the candidate disease developing date extracted in S3022 and the absolute date extracted in S3023 (S3024). In this example, a disease developing date of the patient having the patient code P0 is July 1, 2014 when a value of HbAlc is 6.5% or more according to a definition in the guidelines. However, since a DPP4 inhibitor prescription date extracted in S3023 is May 1, 2013, May 1, 2013 is recorded in a disease developing date management table (shown in FIG. 11) as a disease developing date.

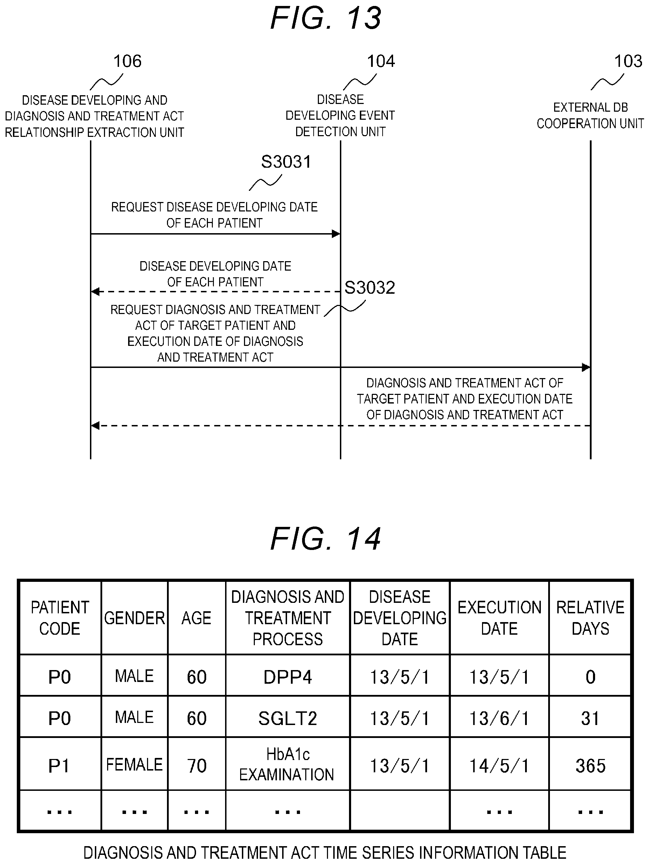

Next, step S303 will be described in detail. FIG. 12 is a flowchart showing step S303 in detail. FIG. 13 is a sequence diagram showing a part of processings (S3031 to S3032) shown in FIG. 12.

First, the disease developing and diagnosis and treatment act relationship extraction unit 106 acquires a disease developing date (for example, the disease developing date management table shown in FIG. 11) of each patient from the disease developing event detection unit 104 (S3031).

Next, the disease developing and diagnosis and treatment act relationship extraction unit 106 accesses the clinical database 102 via the external DB cooperation unit 103, and acquires a diagnosis and treatment act or measure of a target patient and absolute dates of the diagnosis and treatment act or measure (S3032) . In the example shown in FIG. 8, (P0, DPP4, 13/5/1), (P0, SGLT2, 13/6/1), (P1, HbAlc examination, 14/5/1), (P1, body temperature measurement, 14/6/1) are acquired.

Finally, the disease developing and diagnosis and treatment act relationship extraction unit 106 calculates relative days from a disease developing date to an execution date of each diagnosis and treatment act or measure for each patient (S3033). The calculated relative days are recorded in a diagnosis and treatment act time series information table shown in FIG. 14.

Next, two methods to be used in step S304 will be described in detail. A first method focuses on an importance of an early diagnosis and treatment, and a second method focuses on an importance of continuously executing a diagnosis and treatment act or measure.

The first method focuses on the importance of an early diagnosis and treatment. When the disease developing time series information convolution unit 107 generates a data set of explanatory variables, the disease developing time series information convolution unit 107 weights and adds instances of diagnosis and treatment acts or measures executed in an early stage even when the diagnosis and treatment acts are the same. Accordingly, an early diagnosis and an early treatment are emphasized and the data set of explanatory variables obtained by compressing time series components is generated. Specifically, a characteristic amount Xij is calculated for each diagnosis and treatment act or measure j by using the following Formula 1. According to Formula 1, the smaller the relative days from the disease developing date to the execution date, the larger the characteristic amount Xij of the treatment act or measure. The diagnosis and treatment act or measure that contributes to an early diagnosis and an early treatment can be weighted heavily.

[Formula 1]

$$Xij = \sum_t f(t)Rij(t)$$

$$Rij(t) = \begin{cases} 1 \text{ if } Aj \text{ was executed at the related month } t(= Execution\_Month(Aj) - M(i)) \\ 0 \text{ otherwise} \end{cases}$$

$$f(t) = \lfloor t \ divide \ A \rfloor, \qquad (A \text{ is constant.})$$

When M(i) is set as a diabetes developing date of a patient i and a diagnosis and treatment act or measure group A is set as {A1, A2 ... Aj } (for example, A1 = DPP4, A2 = SGLT2), a relative day Rij (t) from the diabetes developing date can be calculated, and the characteristic amount Xij can be calculated by multiplying the relative day Rij (t) with a monotone decreasing function f(t) serving as a weighting element.

The second method focuses on a continuously executed diagnosis and treatment act or measure. When the disease developing time series information convolution unit 107 generates a data set of explanatory variables, the disease developing time series information convolution unit 107 weights and adds instances of continuously executed diagnosis and treatment acts or measures even when the diagnosis and treatment acts are the same. Accordingly, continuity of the diagnosis and treatment acts is emphasized and the data set of explanatory variables obtained by compressing time series components is generated. Specifically, a characteristic amount Xij is calculated for each diagnosis and treatment act or measure j by using the following Formula 2. According to Formula 2, the larger a characteristic amount Xij of a diagnosis and treatment act or measure executed for multiple times at a regular interval, the smaller a characteristic amount Xij of the diagnosis and treatment act or measure executed for multiple times at irregular intervals or executed intensively during one period, and a diagnosis and treatment act or measure whose continuity contributes to improvement of a medical condition can be weighted heavily. In Formula 2, Rij (t) is defined in the same manner as the Rij (t) in Formula 1, and is weighted using a monotone decreasing function f(t) that decreases with elapse of time as an element.

[Formula 2]

$$Xij = \frac{(\sum_t f(t)Rij(t))^2}{\sum_t (f(t)Rij(t))^2}$$

$$Rij(t) = \begin{cases} 1 \text{ if } Aj \text{ was executed at the related month } t(= Execution\_Month(Aj) - M(i)) \\ 0 \text{ otherwise} \end{cases}$$

$$f(t) = \lfloor t \text{ divide } A \rfloor, \qquad (A \text{ is constant.})$$

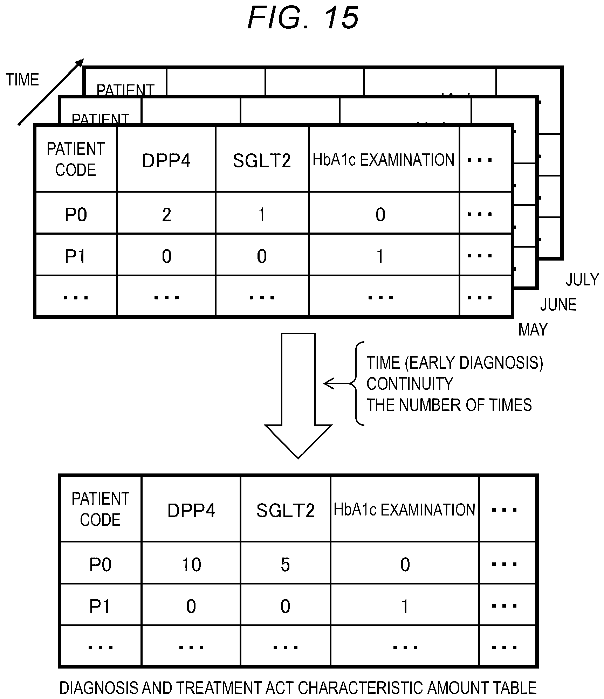

The characteristic amount calculated in step S304 is recorded in a diagnosis and treatment act characteristic amount table shown in FIG. 15. As shown in FIG. 15, the characteristic amount calculated by Formula 2 is generated by compressing time series components in consideration of temporal closeness (an early diagnosis), continuity, and the number of times of diagnosis and treatment acts or measures organized in time series.

Next, step S305 will be described in detail. The evaluation index calculated in step S305 is an index for evaluating medical care quality, and is referred to as a quality indicator or the like. For example, a patient percentage of diabetes patients whose blood glucose control of HbAlc is less than 6.5% is used in the field of diabetes. Therefore, the evaluation index calculation unit 108 acquires clinical data from the clinical database 102 via the external DB cooperation unit 103. The evaluation index calculation unit 108 calculates an evaluation index to be 1 if a value of the HbAlc is 6.5% or more and calculates an evaluation index to be 0 if a value of the HbAlc is less than 6.5%.

Next, step S306 will be described in detail with reference to FIG. 16.

First, the diagnosis and treatment effect extraction unit 109 acquires the characteristic amount of a diagnosis and treatment act or measure (a diagnosis and treatment act characteristic amount table shown in FIG. 15) generated by the disease developing time series information convolution unit 107 (S3061).

Next, an initial value of an effect characteristic amount is acquired via the evaluation index calculation unit 108 (S3062). In the example shown in FIG. 6, since a target period is specified from year 2013 in the condition setting and processing result display screen (shown in FIG. 6), an evaluation index of year 2013 is acquired. As described above, an initial value of a characteristic amount of an index value may be used or may not be used.

Then, a result of S3061 and a result of S3062 are integrated to create a characteristic amount vector for each patient (S3063). The characteristic amount vector to be used as an explanatory variable is generated in this manner, so that an existing selection method can be used and easily implemented in the system.

Next, a final result of the effect characteristic amount is acquired via the evaluation index calculation unit 108 (S3064). In the example shown in FIG. 6, since the target period is specified until year 2016 in the condition setting and processing result display screen (shown in FIG. 6), an evaluation index of year 2016 is acquired.

Finally, a characteristic that affects a final result of the effect characteristic amount is selected from the characteristic amount vector generated in S3063 (S3065). Specifically, the characteristic is selected by setting a characteristic selection method such as a linear regression model (for example, binary logistic regression) and a nonlinear model (for example, random forest and gradient boost) with a characteristic amount vector output in S3063 as an explanatory variable and setting a variable output in S3064 as an objective variable.

The screen configuration processing unit 114 generates display data for displaying, on the display unit 116, a highly effective diagnosis and treatment act or measure calculated according to such a procedure. For example, a calculation result is displayed in the processing result presentation region 502 as shown in FIG. 21. According to FIG. 21, it can be seen that a diagnosis and treatment act or measure that most greatly affects an evaluation index is to take the SGLT2 inhibitor.

In the processing (S304) executed by the disease developing time series information convolution unit 107, with respect to the processing of "extracting an effective diagnosis and treatment act or measure in consideration of time series components in addition to whether the diagnosis and treatment act or measure is executed", when a data set of explanatory variables is generated, the data set of explanatory variables is generated in consideration of a viewpoint of an early diagnosis and an early treatment while compressing the time series components by weighting and adding instances of events executed in an early stage even when the acts are the same. The processing in step S304 can be implemented by simultaneously introducing processings in step S302 and step S303 when a concept of "early execution" is introduced.

Next, step S307 will be described in detail with reference to FIG. 17.

First, the target disease total cost calculation unit 110 acquires a disease developing date (for example, the disease developing date management table shown in FIG. 11) for each patient from the disease developing event detection unit 104 (S3071).

Next, the target disease total cost calculation unit 110 accesses the medical cost database 100 via the external DB cooperation unit 103, and acquires medical costs after a disease developing date for a target patient (S3072). For example, when referring to the disease developing date management table (shown in FIG. 11), since a diabetes developing date of the patient having the patient code P1 is May 1, 2013, medical costs after May 1, 2013 are acquired from the medical cost database 100. That is, medical costs of the patient having the patient code P1 after May 1, 2013 (that is, June 1, 2013, May 1, 2014, and June 1, 2014) are acquired from a medical cost table (shown in FIG. 19).

Finally, the target disease total cost calculation unit 110 calculates total medical costs after the disease developing date for each patient (S3073). The total medical costs calculated at this time may be (1) a sum of all medical costs after the disease developing date, (2) a sum of medical costs of a corresponding disease name after the disease developing date, and (3) a sum of medical costs including that of a disease (for example, hyperlipidemia which is a complication of diabetes) related to a specified disease after the disease developing date. When medical costs of a related disease are totalized, a disease name related to the corresponding disease may be acquired by referring to a related disease name table shown in FIG. 18. Medical costs for treating a complication or the like can be totalized by totalizing the medical costs of a related disease name.

As shown in FIG. 18, a main disease name and a related disease name are recorded in association with each other in the related disease name table. That is, a patient who has developed a main disease may develop a related disease. For example, a diabetes patient may develop hypertension, dyslipidemia, nephropathy, or the like. Medical costs are totalized with reference to the related disease name table, so that medical costs for treating a complication or the like can be included and totalized, and the medical costs can be accurately totalized by excluding irrelevant medical costs

(for example, a diabetes patient needs to pay for a fracture treatment).

The related disease name table may be provided in the target disease total cost calculation unit 110, or may be acquired from an external database.

As shown in FIG. 19, a medical cost table stored in the medical cost database 100 includes a patient attribute (a patient code or the like), a disease name, an execution date, and a medical cost. The medical cost may be recorded in an amount of money, or may be recorded in any unit that can be converted into an amount of money such as the number of insurance points . The medical cost table may also record costs of a measure (for example, health diagnosis, and healthcare guidance) other than a diagnosis and treatment act.

Next, step S308 will be described in detail with reference to FIG. 20.

First, based on whether the diagnosis and treatment act selected in the condition setting and processing result display screen (shown in FIG. 21) is executed, the medical care economic evaluation calculation unit 111 divides target patients into two groups by referring to the diagnosis and treatment act data (shown in FIG. 8) (S3081). According to the diagnosis and treatment act data shown in FIG. 8, a patient having the patient code P0 who has taken the SGLT2 inhibitor is classified into an execution group. On the other hand, a patient having the patient code P1 who has not taken the SGLT2 inhibitor is classified into a non-execution group. Among the diagnosis and treatment act data, a diagnosis and treatment act after a disease developing date may be referred to, or all diagnosis and treatment acts may be referred to.

Next, the medical care economic evaluation calculation unit 111 calculates an average value of total medical costs for each group (S3082). Specifically, the medical care economic evaluation calculation unit 111 acquires medical costs of a specified disease name that are totalized for each patient in step S307 and calculates an average value of total medical costs for a patient in each group. Instead of the average value, other statistical processings (for example, calculating a maximum value, a minimum value, a mode value, and a variance) may be performed depending on applications.

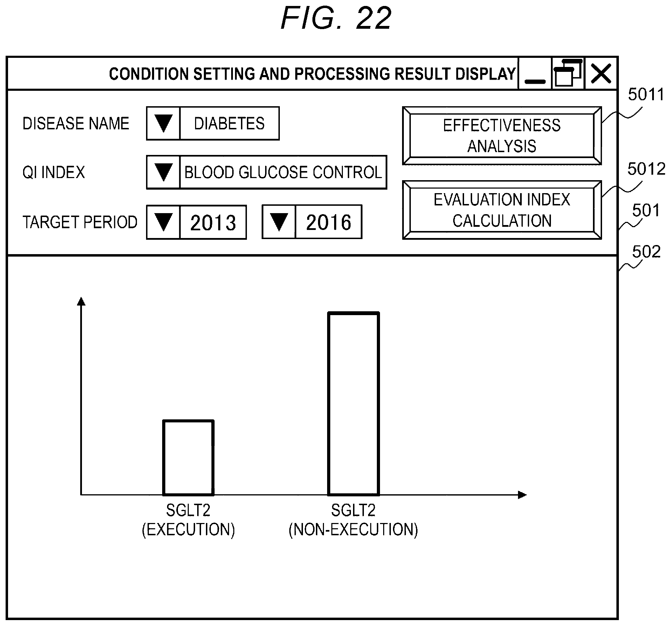

For example, when the SGLT2 inhibitor is selected in the condition setting and processing result display screen (shown in FIG. 21), medical costs are displayed in the condition setting and processing result display screen (shown in FIG. 22) in a manner in which a group of patients who takes the SGLT2 inhibitor and a group of patients who do not take the SGLT2 inhibitor can be compared with each other.

FIG. 21 is a diagram showing an example of a condition setting and processing result display screen displayed by the display unit 116 in step S3081.

Similar to the screen shown in FIG. 6, the condition setting and processing result display screen shown in FIG. 21 includes the condition setting region 501 and the processing result presentation region 502. The condition setting region 501 displays the "effectiveness analysis" button 5011 that is operated to analyze effectiveness of a diagnosis and treatment act, the "evaluation index calculation" button 5012 that is operated to evaluate an economic value of a diagnosis and treatment act and measure, and a pull-down selection input field for setting an analysis condition. In the example shown in FIG. 21, a condition is set for totalizing medical costs of a diagnosis and treatment act or measure having a high effect of affecting blood glucose control by using data of a diabetes patient from year 2013 to year 2016.

As described above, the processing result presentation region 502 displays a highly effective diagnosis and treatment act or measure. According to FIG. 21, it can be seen that a most highly effective treatment act or measure is to take the SGLT2 inhibitor. A "display" button may be provided in the processing result presentation region 502. Although a processing of analyzing a diagnosis and treatment act selected by an alternative selection in the selection field is started in the example described above, a plurality of diagnosis and treatment acts can be selected by providing the "display" button. Therefore, when there is a significant difference in diagnosis and treatments due to a combination of a plurality of diagnosis and treatment acts, effects of the diagnosis and treatment acts (that is, medical costs) can be accurately analyzed. Here, the SGLT2 inhibitor can be selected because a diagnosis and treatment act affecting the quality was automatically selected in S306. In the medical care field, it is unacceptable to evaluate everything by an economic evaluation alone, and it is strongly required to consider medical care quality at the same time. Therefore, an economic evaluation of a diagnosis and treatment act or measure that affects medical care quality can be performed by combining with S306.

FIG. 22 is a diagram showing an example of a condition setting and processing result display screen displayed by the display unit 116 after the processing in step S308 is ended.

Similar to the screen shown in FIG. 21, a condition setting and processing result display screen shown in FIG. 22 includes the condition setting region 501 and the processing result presentation region 502. The screen configuration processing unit 114 generates display data for displaying medical costs in the display unit 116 in a manner in which the execution group and the non-execution group can be compared with each other, and displays the display data in the processing result presentation region 502.

Next, step S309 will be described in detail with reference to FIG. 23.

First, the target patient medical care act extraction unit 112 receives a process having a high importance degree calculated in S306 among medical care processes for a patient to be analyzed (S3091).

Next, for the diagnosis and treatment processes extracted in S303, the target patient medical care act extraction unit 112 calculates relative days starting from when the process is received in S3091 for each patient (S3092).

Then, the target patient medical care act extraction unit 112 calculates an average value of the relative days extracted in S3092 for each diagnosis and treatment process (S3093). The target patient medical care act extraction unit 112 extracts a diagnosis and treatment process whose average value of the relative days calculated in S3093 is equal to or larger than a preset threshold (S3094). The threshold in S3094 may be set to a period required to prepare a diagnosis and treatment. The threshold may be set as an average value of relative days, or may be set using a value obtained by adding a variance to or subtracting a variance from the average value. In S3093 and S3094, statistical values (such as a median value) other than the average value may be used.

In step S3094, a diagnosis and treatment process whose average value of the relative days is equal to or less than a preset threshold may be extracted.

Next, the candidate extraction unit 113 extracts pairs of the economic evaluation generated in S308 and a diagnosis and treatment act by using the diagnosis and treatment process extracted in S3094 as a key (S3095).

Finally, the candidate extraction unit 113 extracts, from the pairs extracted in S3095, a pair in which an economic evaluation is in an upper level (S3096). The candidate extraction unit 113 may extract a pair in which an importance degree is in an upper level, or may extract a pair in which an importance degree and an economic evaluation are integrated and both are in an upper level.

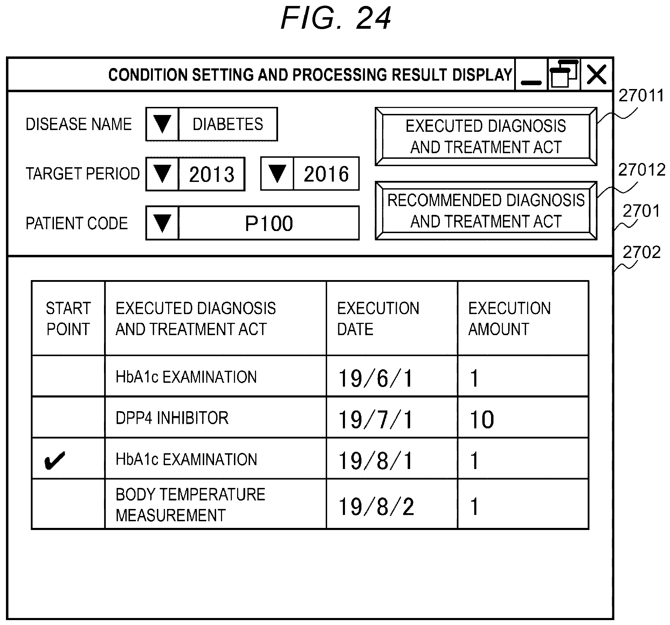

FIG. 24 is a diagram showing an example of a condition setting and processing result display screen displayed by the display unit 116 in step S3091.

The condition setting and processing result display screen shown in FIG. 24 includes a condition setting region 2701 and a starting point setting input region 2702. The condition setting region 2701 displays an "executed diagnosis and treatment act" button 27011 that is operated to analyze effectiveness of an executed diagnosis and treatment act, a "recommended diagnosis and treatment act" button 27012 that is operated to receive presentation of an effective diagnosis and treatment act, and a pull-down selection input field for setting an analysis condition. In the example shown in FIG. 24, a condition is set for receiving presentation of an effective diagnosis and treatment act for a diabetes patient having a patient code P100 by using data of the diabetes patient from year 2013 to year 2016.

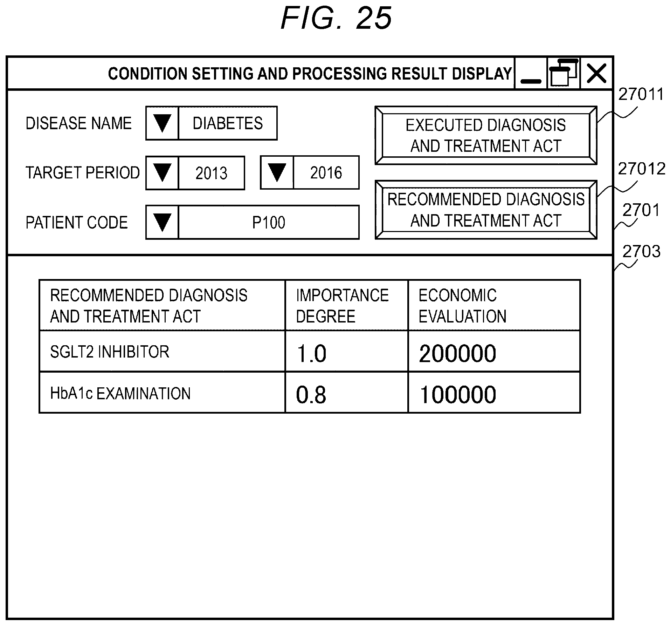

As described above, the starting point setting input region 2702 displays a highly effective diagnosis and treatment act. That is, when a user operates the "executed diagnosis and treatment act" button 27011, diagnosis and treatment processes extracted in step S303 by the target patient medical care act extraction unit 112 are displayed in the starting point setting input region 2702. Then, when the user selects a process having a high importance degree to be a starting point from the displayed diagnosis and treatment processes (S3091) and operates the "recommended treatment act" button 27012, the screen shown in FIG. 25 is displayed and an effective diagnosis and treatment act is recommended.

FIG. 25 is a diagram showing an example of a condition setting and processing result display screen displayed by the display unit 116 after the processing in step S309 is ended.

The condition setting and processing result display screen shown in FIG. 25 includes the condition setting region 2701 and a processing result presentation region 2703. The condition setting region 2701 is the same as that in the condition setting and processing result display screen shown in FIG. 24.

As described above, the processing result presentation region 2703 displays a highly effective diagnosis and treatment act. According to FIG. 25, it can be seen that a highly effective diagnosis and treatment act or measure is to take the SGLT2 inhibitor and the HbAlc examination, and the SGLT2 inhibitor has a high effect in terms of both importance degree and economic evaluation.

Hereinafter, modifications of the embodiment of the invention will be described.

<First Modification>

Although one diagnosis and treatment act selected by the user is economically evaluated in the embodiment described above, a plurality of diagnosis and treatment acts are economically evaluated in a first modification.

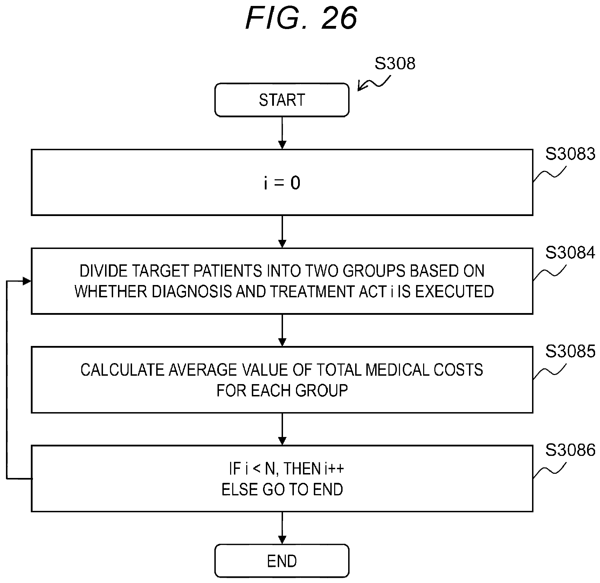

FIG. 26 is a flowchart showing step S308 in detail according to the first modification.

First, the medical care economic evaluation calculation unit 111 selects one diagnosis and treatment act i (S3083). The diagnosis and treatment acts may be selected from the disease developing knowledge data (shown in FIG. 10) or the diagnosis and treatment act data (shown in FIG. 8) in a manner in which the diagnosis and treatment acts i for disease names to be analyzed are not repeated. A table obtained by extracting in advance diagnosis and treatment acts for the disease names to be analyzed from the disease developing knowledge data (shown in FIG. 10) or the diagnosis and treatment act data (shown in FIG. 8) may be created. The diagnosis and treatment acts i may be selected one by one from the created table in step S3083.

Next, based on whether the diagnosis and treatment acts i selected in step S3083 are executed, the medical care economic evaluation calculation unit 111 divides target patients into two groups by referring to the diagnosis and treatment act data (shown in FIG. 8) (S3084) . Among the diagnosis and treatment act data, a diagnosis and treatment act after a disease developing date may be referred to, or all diagnosis and treatment acts may be referred to.

Then, the medical care economic evaluation calculation unit 111 calculates an average value of total medical costs for each group (S3085). Specifically, the medical care economic evaluation calculation unit 111 acquires medical costs of a specified disease name that are totalized for each patient in step S307 and calculates an average value of total medical costs for a patient in each group. Instead of the average value, other statistical processings (for example, calculating a maximum value, a minimum value, a mode value, and a variance) may be performed depending on applications.

Next, if a parameter i for controlling the diagnosis and treatment acts is smaller than a total number N of the diagnosis and treatment acts, the medical care economic evaluation calculation unit 111 returns the processing to step S3084 and executes the processing for a subsequent diagnosis and treatment act. On the other hand, if the parameter i is equal to or larger than the total number N of the diagnosis and treatment acts, analyses for all the diagnosis and treatment acts are completed and the processing is ended (S3086).

In addition to a loop of the parameter i for the diagnosis and treatment acts, a loop for disease names may be provided, and an average value of total medical costs of a plurality of disease names may be calculated. In this case, an average value of total medical costs of all disease names may be calculated, or an average value of total medical costs of two or more selected disease names may be calculated.

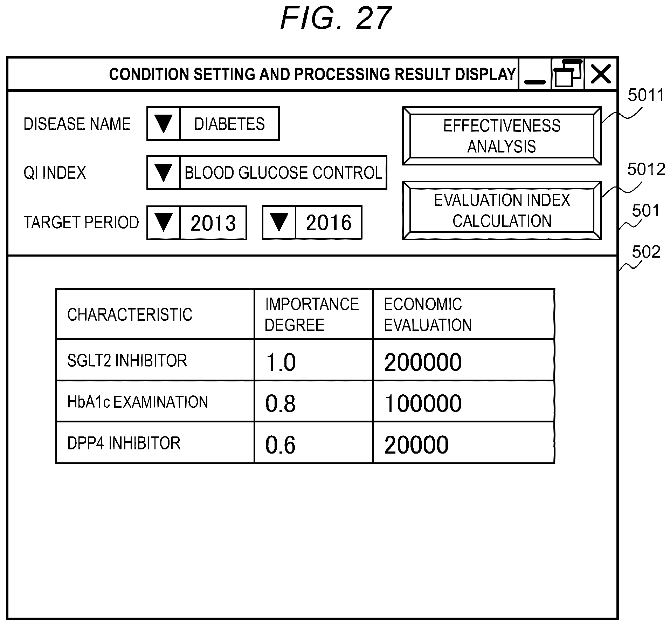

FIG. 27 is a diagram showing an example of a condition setting and processing result display screen displayed by the display unit 116 after the processing in step S308 is ended according to the first modification.

Similar to the screen shown in FIG. 6, the condition setting and processing result display screen shown in FIG. 27 includes the condition setting region 501, the processing result presentation region 502. The condition setting region 501 displays the "effectiveness analysis" button 5011 that is operated to analyze effectiveness of a diagnosis and treatment act, the "evaluation index calculation" button 5012 that is operated to evaluate an economic value of a diagnosis and treatment act or measure, and a pull-down selection input field for setting an analysis condition. In the example shown in FIG. 27, a condition is set for totalizing medical costs of a diagnosis and treatment act having a high effect of affecting blood glucose control by using data of a diabetes patient from year 2013 to year 2016.

The processing result presentation region 502 displays an economic evaluation of a highly effective diagnosis and treatment act or measure. According to FIG. 27, it can be seen that a diagnosis and treatment act or measure that is highly effective (that is, an importance degree is high in FIG. 27) as a diagnosis and treatment act is to take the SGLT2 inhibitor and the diagnosis and treatment act or measure of taking the SGLT2 inhibitor has highest economic effect. An economic evaluation may be displayed in an amount of money, or may be displayed in any unit that can be converted into an amount of money such as the number of insurance points.

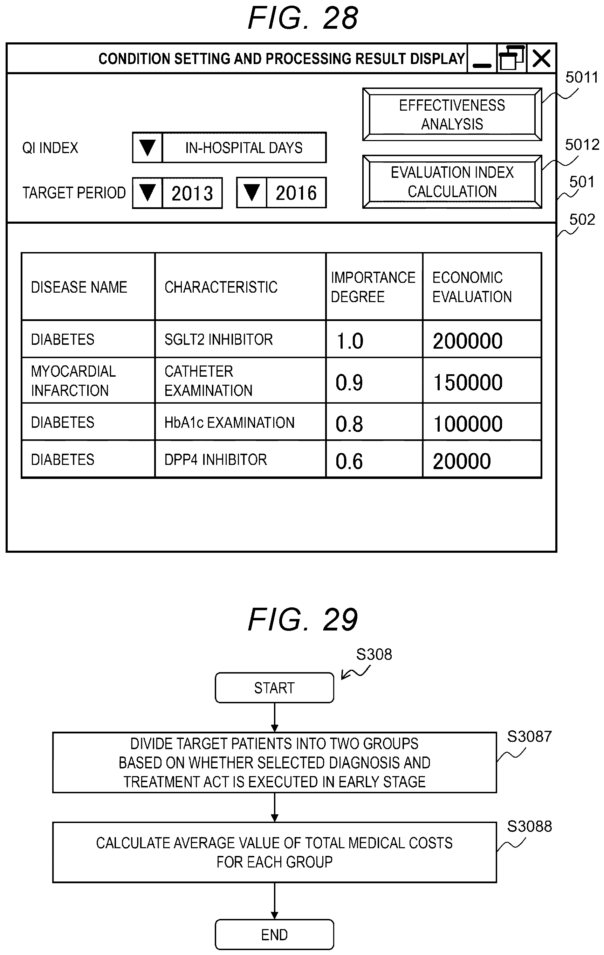

FIG. 28 is a diagram showing another example of a condition setting and processing result display screen of the first modification displayed by the display unit 116 after the processing in step S308 is ended according to the first modification. The condition setting and processing result display screen shown in FIG. 28 is displayed by calculating an average value of total medical costs of diagnosis and treatment acts for all disease names in processings shown in FIG. 26.

Similar to the screen shown in FIG. 27, the condition setting and processing result display screen shown in FIG. 28 includes the condition setting region 501, the processing result presentation region 502. The condition setting region 501 displays an "effectiveness analysis" button 5011 that is operated to analyze effectiveness of a diagnosis and treatment act, an "evaluation index calculation" button 5012 that is operated to evaluate an economic value of a diagnosis and treatment act or measure, and a pull-down selection input field for setting an analysis condition. In the example shown in FIG. 28, a condition is set for totalizing medical costs of a diagnosis and treatment act having a high effect of affecting in-hospital days by using data from year 2013 to year 2016.

The processing result presentation region 502 displays an economic evaluation of a highly effective diagnosis and treatment act or measure. According to FIG. 28, it can be seen that as a diagnosis and treatment act or measure that affects in-hospital days, a diagnosis and treatment act that has a high effect (that is, an importance degree is high in FIG. 28) is to take the SGLT2 inhibitor for treating diabetes and the diagnosis and treatment act or measure of taking the SGLT2 inhibitor also has highest economic effect. An economic evaluation may be displayed in an amount of money, or may be displayed in any unit that can be converted into an amount of money such as the number of insurance points.

Although medical costs of all disease names are totalized on the screen shown in FIG. 28, an "add" button may be provided on the screen shown in FIG. 27 and a plurality of disease names may be selected. Medical costs of the selected disease names may be totalized to display economic evaluations. Further, only medical costs of related disease names may be totalized to display economic evaluations by using the related disease name table shown in FIG. 18.

Economic evaluations of a plurality of disease names may be displayed in one table as shown in FIG. 28. Alternatively, an economic evaluation of each disease name may be displayed in a table.

As described above, economic evaluations of a plurality of diagnosis and treatment acts can be viewed according to the first modification, and a diagnosis and treatment act having a high economic effect can be known.

<Second Modification>

Although patients are divided into two groups based on whether a specified diagnosis and treatment act is executed and medical costs of the two groups are compared with each other according to the embodiment described above, patients are divided into two groups according to an execution period of a specified diagnosis and treatment act and medical costs of the two groups are compared with each other according to a second modification.

FIG. 29 is a flowchart showing step S308 in detail according to the second modification.

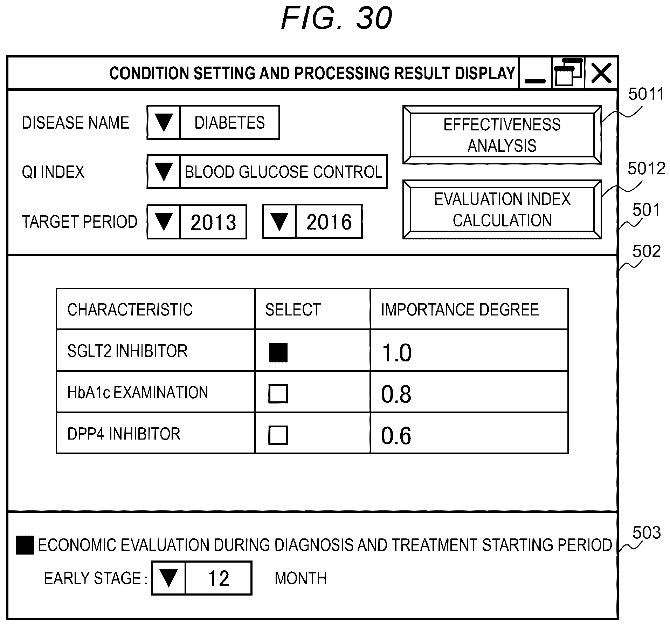

First, based on whether the diagnosis and treatment act selected on the condition setting and processing result display screen (shown in FIG. 21) is executed in an early stage, the medical care economic evaluation calculation unit 111 divides target patients into two groups (including an early execution group and a late execution group) by referring to the diagnosis and treatment act data (shown in FIG. 8) and the disease developing date management table (shown in FIG. 11) (S3087). According to the diagnosis and treatment act data shown in FIG. 8, the patient having the patient code P0 developed diabetes on May 1, 2013 and took the SGLT2 inhibitor on June 1, 2013. Here, an early stage determination criterion is set to 12 months in a condition setting and processing result display screen (shown in FIG. 30) . Since it is 1 month from disease developing to administration of the SGLT2 inhibitor, the patient having the patient code P0 is classified into the early execution group. On the other hand, since the patient having the patient code P1 did not take the SGLT2 inhibitor, the patient is not included in the early execution group or the late execution group. Among diagnosis and treatment act data, a diagnosis and treatment act after a disease developing date may be referred to, or all diagnosis and treatment acts may be referred to.

Next, the medical care economic evaluation calculation unit 111 calculates an average value of total medical costs for each group (S3088). Specifically, the medical care economic evaluation calculation unit 111 acquires medical costs of a specified disease name that are totalized for each patient in step S307 and calculates an average value of total medical costs for a patient in each group. Instead of the average value, other statistical processings (for example, calculating a maximum value, a minimum value, a mode value, and a variance) may be performed depending on applications.

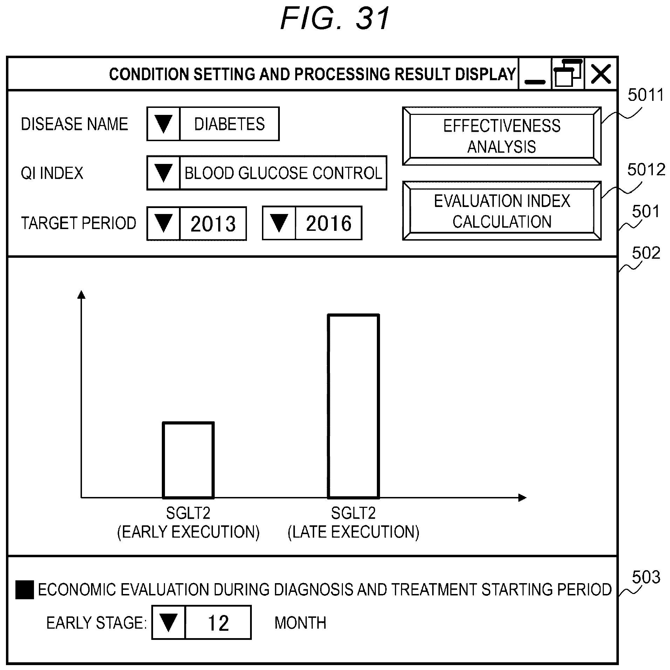

For example, when the SGLT2 inhibitor is selected and an early stage determination criterion is set to 12 months in the condition setting and processing result display screen (shown in FIG. 30), medical costs are displayed on a condition setting and processing result display screen (shown in FIG. 31) in a manner in which medical costs of a group of patients who takes the SGLT2 inhibitor within 12 months after disease developing and medical costs of a group of patients who do not take the SGLT2 inhibitor can be compared with each other.

FIG. 30 is a diagram showing an example of a condition setting and processing result display screen displayed by the display unit 116 in step S3081.

Similar to the screen shown in FIG. 6, the condition setting and processing result display screen shown in FIG. 30 includes the condition setting region 501, the processing result presentation region 502, and the time setting region 503. The condition setting region 501 displays an "effectiveness analysis" button 5011 that is operated to analyze effectiveness of a diagnosis and treatment act, an "evaluation index calculation" button 5012 that is operated to evaluate an economic value of a diagnosis and treatment act or measure, and a pull-down selection input field for setting an analysis condition. In the example shown in FIG. 30, a condition is set for totalizing medical costs of a diagnosis and treatment act having a high effect of affecting blood glucose control by using data of a diabetes patient from year 2013 to year 2016.

As described above, the processing result presentation region 502 displays a highly effective diagnosis and treatment act or measure. According to FIG. 30, it can be seen that a most highly effective diagnosis and treatment act or measure is to take the SGLT2 inhibitor. A "display" button may be provided in the processing result presentation region 502. Although a processing of analyzing a diagnosis and treatment act selected by an alternative selection in the selection field is started in the example described above, a plurality of diagnosis and treatment acts can be selected by providing the "display" button. Therefore, when there is a significant difference in diagnosis and treatments due to a combination of a plurality of diagnosis and treatment acts, an effect of the diagnosis and treatment acts (that is, medical costs) can be accurately analyzed.

An early stage determination criterion (a period from disease developing to when a diagnosis and treatment act is taken) for dividing patients into two groups is set in the time setting region 503. In the example shown in FIG. 30, a condition for determining "an early stage" when a diagnosis and treatment act is executed within 12 months after disease developing is set.

FIG. 31 is a diagram showing an example of a condition setting and processing result display screen displayed by the display unit 116 after the processing in step S308 is ended.

Similar to the screen shown in FIG. 30, the condition setting and processing result display screen shown in FIG. 31 includes the condition setting region 501, the processing result presentation region 502, and the time setting region 503. The screen configuration processing unit 114 generates display data for displaying medical costs on the display unit 116 in a manner in which the early execution group and the late execution group can be compared with each other, and displays the display data in the processing result presentation region 502.

As described above, in the second modification, an economic effect of executing a diagnosis and treatment act in an early stage can be known. A treatment period when the economic effect occurs can be known by setting a period as a parameter and analyzing the parameter.

<Third Modification>

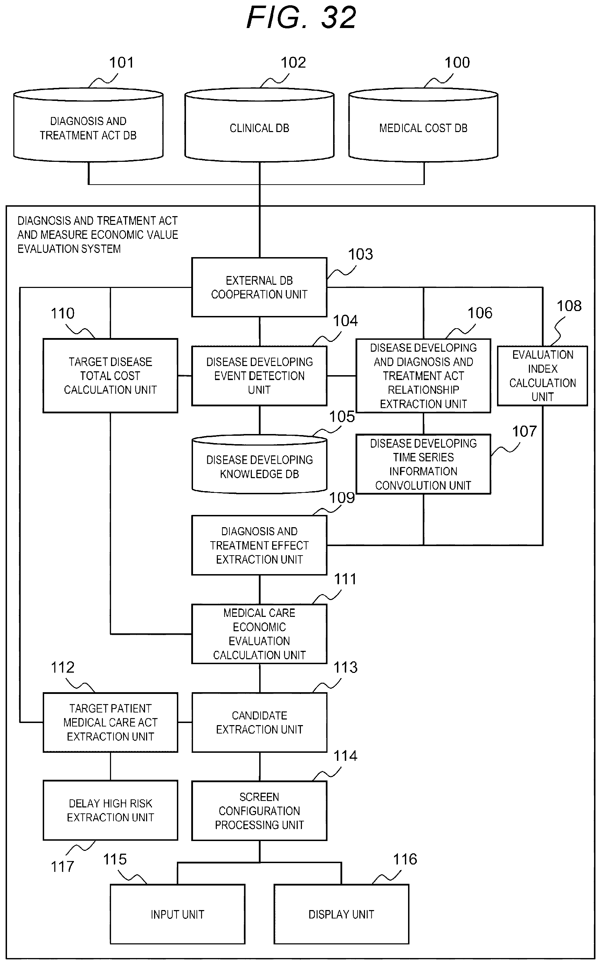

FIG. 32 is a configuration diagram showing a diagnosis and treatment act and measure economic value evaluation system according to the present modification.

The system includes the external DB cooperation unit 103, the disease developing event detection unit 104, the disease developing knowledge database 105, the disease developing and diagnosis and treatment act relationship extraction unit 106, the disease developing time series information convolution unit 107, the evaluation index calculation unit 108, the diagnosis and treatment effect extraction unit 109, the target disease total cost calculation unit 110, the medical care economic evaluation calculation unit 111, the target patient medical care act extraction unit 112, the candidate extraction unit 113, the screen configuration processing unit 114, the input unit 115, the display unit 116, and a delay high risk extraction unit 117. Since the configuration other than the configuration of the delay high risk extraction unit 117 is the same as that of the diagnosis and treatment act and measure economic value evaluation system according to the first embodiment described above in FIG. 2, descriptions thereof will be omitted.

The delay high risk extraction unit 117 extracts a medical care process having a high delay risk. The process having a high delay risk is a medical care process whose variance of relative days is large. That is, when a variance of relative days is large, it is most likely that a medical care process cannot be provided at medically appropriate timing due to a problem such as resource allocation. Therefore, when the medical care process is delayed, the effect may be lowered and the medical care process is required to be executed in an early stage.

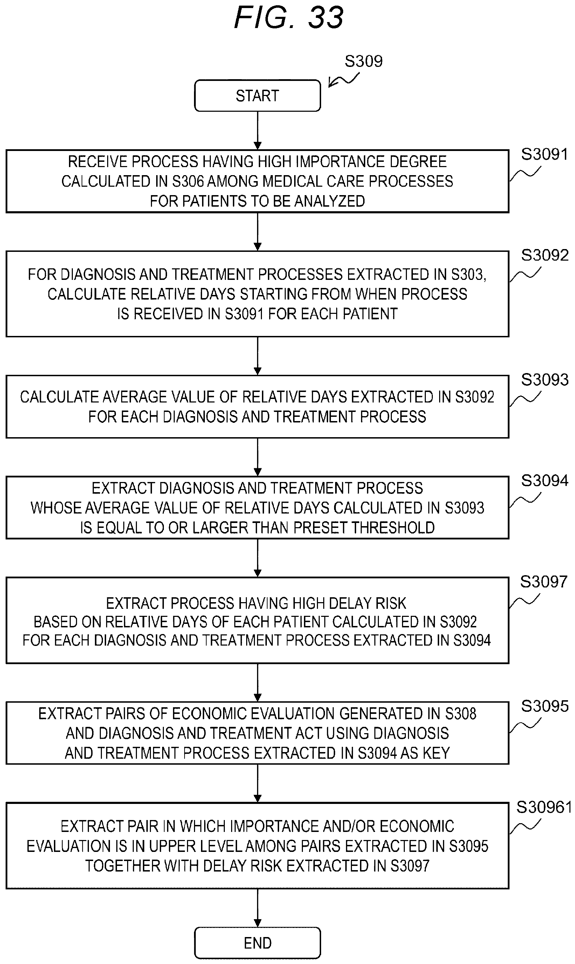

Hereinafter, step S309 will be described in detail with reference to FIG. 33.

First, among medical care processes for patients to be analyzed, the target patient medical care act extraction unit 112 receives a process having a high importance degree calculated in S306 (S3091). Among diagnosis and treatment processes extracted in S303, the target patient medical care act extraction unit 112 calculates, for each patient, relative days starting from when the process is received in S3091 (S3092) . The target patient medical care act extraction unit 112 calculates an average value of the relative days extracted in S3092 for each diagnosis and treatment process (S3093). The target patient medical care act extraction unit 112 extracts a diagnosis and treatment process whose average value of relative days calculated in S3093 is equal to or larger than a preset threshold (S3094) . Since the processings from S3091 to S3094 that are executed by the target patient medical care act extraction unit 112 are the same as the processing in step S309 described in FIG. 23 according to the first embodiment, detailed descriptions will be omitted.

Thereafter, the delay high risk extraction unit 117 extracts a process having a high delay risk based on relative days of each patient in S3092 for each diagnosis and treatment process extracted in S3094 (S3097) . Specifically, the average value of relative days extracted in S3092 is compared with a predetermined threshold to determine whether a delay risk of a medical care process is high. Whether a medical care process has a delay risk is determined according to magnitude of the variance of relative days in step S3094. Alternatively, whether a medical care process has a delay risk may be determined by numeralizing the delay risk (for example, the delay risk has numerical values of 0 to 100).

Next, the candidate extraction unit 113 extracts pairs of the economic evaluation generated in S308 and a diagnosis and treatment act by using the diagnosis and treatment process extracted in S3094 as a key (S3095).

Finally, together with the delay risk extracted in S3097, the candidate extraction unit 113 extracts a pair in which an economic evaluation is in an upper level among the pairs extracted in S3095 (S30961). The candidate extraction unit 113 may extract a pair in which an importance degree is in an upper level, or may extract a pair in which an importance degree and an economic evaluation are integrated and both are in an upper level. The delay risk calculated in S3097 is presented together with an economic evaluation and a diagnosis and treatment act, and may be considered in calculation of an importance degree.

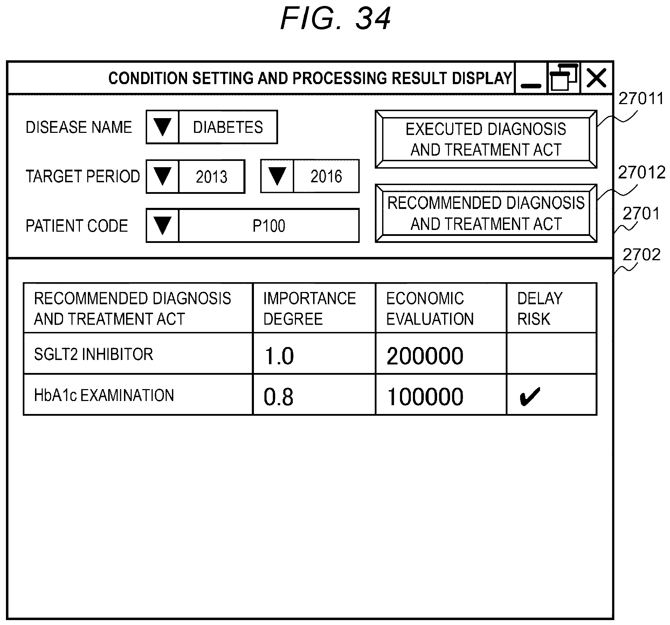

FIG. 34 is a diagram showing an example of a condition setting and processing result display screen displayed by the display unit 116 after the processing in step S309 is ended.

The condition setting and processing result display screen shown in FIG. 34 includes the condition setting region 2701 and the processing result presentation region 2703. The condition setting region 2701 is the same as those in the condition setting and processing result display screens shown in FIGS. 24 and 25.

As described above, the processing result presentation region 2703 displays a highly effective diagnosis and treatment act and whether there is a delay risk. According to FIG. 34, it can be seen that a highly effective diagnosis and treatment act or measure is to take the SGLT2 inhibitor and the HbAlc examination, and the SGLT2 inhibitor has a high effect in terms of both importance degree and economic evaluation.

As described above, the analysis system according to the embodiment of the invention includes the input unit 115 that receives medical care act data (the diagnosis and treatment act database 101), clinical data (the clinical database 102) including an examination result of a patient, medical cost data (the medical cost database 100), and medical care act and clinical data of a patient to be analyzed, the disease developing event detection unit 104 that detects a disease developing event, the disease developing and diagnosis and treatment act relationship extraction unit 106 that extracts a correlation between a detected disease developing event and a medical care act based on time series information, the evaluation index calculation unit 108 that calculates an index value indicating medical care quality based on the received medical care act data and clinical data, the target disease total cost calculation unit 110 that calculates costs of a medical care act that relate to a disease name to be analyzed and are generated after a period of the detected disease developing event by using the medical cost data, and an extraction unit (including the target patient medical care act extraction unit 112 and the candidate extraction unit 113) that extracts a recommended medical care act for the patient to be analyzed by using at least one of the calculated index value and the calculated costs, the received medical care act and clinical data of the patient to be analyzed, and a correlation extracted by the disease developing and diagnosis and treatment act relationship extraction unit 106. Therefore, an effective diagnosis and treatment act can be recommended for each patient.

The extraction unit includes the target patient medical care act extraction unit 112 that extracts future candidate medical care acts for the patient to be analyzed by using the received medical care act and clinical data of the patient to be analyzed and the correlation extracted by the disease developing and diagnosis and treatment act relationship extraction unit 106, and the candidate extraction unit 113 that extracts a recommended medical care act for the patient from the extracted future candidate medical care acts by using at least one of the calculated index value and the calculated costs . Therefore, an effective diagnosis and treatment act can be accurately recommended for each patient.

The input unit 115 receives a selection of a medical care act serving as a starting point of a relative period from medical care acts executed on the presented patient to be analyzed. Based on a comparison result between a predetermined threshold and a statistical value of relative periods between the selected medical care act serving as the starting point and an extracted candidate medical care act, the target patient medical care act extraction unit 112 extracts a future candidate medical care act. Therefore, an effective diagnosis and treatment act can be accurately recommended for each patient.

Among the medical care acts executed on the patient to be analyzed, the input unit 115 presents a medical care act having a high importance degree. Therefore, an effective diagnosis and treatment act can be recommended for each patient.

The screen configuration processing unit 114 generates screen data for outputting the medical care acts that are executed on the patient to be analyzed and are received by the input unit 115 and a medical care act extracted by the extraction unit. Therefore, an effective diagnosis and treatment act can be recommended for each patient.

Since the screen configuration processing unit 114 generates screen data for outputting a variance of relative periods, an appropriate execution time of the recommended diagnosis and treatment act can be known.

Since the target patient medical care act extraction unit 112 sets the threshold by using the statistical value of the relative periods, an effective diagnosis and treatment act can be recommended for each patient.

Since the screen configuration processing unit 114 generates screen data in a manner in which medical costs of the extracted medical care acts can be compared between a group having a long relative period and a group having a short relative period, an effective diagnosis and treatment act can be recommended for each patient.

<Other Aspects of Invention Disclosed in Present Specification>

In respect of the invention disclosed in the present description, typical aspects of the invention other than those described in the claims are as follows.

(1) An analysis system that analyzes an effect of a diagnosis and treatment act and measure and is implemented by a computer including an arithmetic device that executes a predetermined processing and a storage device connected to the arithmetic device, the analysis system including:

an input unit that receives an analysis condition;
an event detection unit that extracts a disease developing event; and
a cost calculation unit that calculates costs of a diagnosis and treatment act that relate to a disease name to be analyzed and are generated after a period of the disease developing event extracted by the event detection unit.

(2) The analysis system according to aspect (1), wherein the cost calculation unit calculates costs of a diagnosis and treatment act that relate to a disease name the same as the disease name to be analyzed and are generated after the period of the disease developing event.

(3) The analysis system according to aspect (1), wherein the cost calculation unit specifies a diagnosis and treatment act for a disease name related to the disease name to be analyzed by referring to related disease name information in which a related disease name is stored, and calculates costs of the specified diagnosis and treatment act and costs of a diagnosis and treatment act for the disease name to be analyzed that are generated after the period of the disease developing event.

(4) The analysis system according to aspect (1), further including:
an economic evaluation calculation unit that totalizes medical costs of the disease name to be analyzed by dividing patients into an execution group in which a specified diagnosis and treatment act is executed and a non-execution group in which the specified diagnosis and treatment act is not executed.

(5) The analysis system according to aspect (4), wherein the economic evaluation calculation unit totalizes medical costs of the disease name to be analyzed for each of a plurality of diagnosis and treatment acts by dividing patients into an execution group of a corresponding diagnosis and treatment act and an non-execution group of the diagnosis and treatment act.

(6) The analysis system according to aspect (5), further including:

a screen configuration processing unit that generates screen display data to be output to a display device, wherein the economic evaluation calculation unit totalizes medical costs of all disease names to be analyzed or a selected disease name to be analyzed for each of a plurality of diagnosis and treatment acts by dividing patients into an execution group of a corresponding diagnosis and treatment act and a non-execution group of the diagnosis and treatment act, and

the screen configuration processing unit generates display data for displaying economic evaluations of the plurality of diagnosis and treatment acts in a descending order of a difference of totalized medical costs between the execution group and the non-execution group.

(7) The analysis system according to aspect (1), further including:
an economic evaluation calculation unit that totalizes medical costs of the disease name to be analyzed by dividing patients into a group in which a specified diagnosis and treatment act is executed within a predetermined period from the disease developing event and a group in which the specified diagnosis and treatment act is executed after the predetermined period.

(8) The analysis system according to aspect (1), further including:

a relationship extraction unit that calculates a time series relationship between a period of the disease developing event extracted by the event detection unit and an execution period of the diagnosis and treatment act and measure;

a characteristic generation unit that generates a characteristic amount of the diagnosis and treatment act and measure in consideration of the time series relationship based on the time series relationship calculated by the relationship extraction unit and an execution amount of the diagnosis and treatment act and measure;

an index calculation unit that calculates an index value indicating medical care quality based on clinical data including a history of the diagnosis and treatment act and measure and an examination result of a patient; and

an effect extraction unit that extracts a diagnosis and treatment act and measure having a good index value by setting the characteristic amount of the diagnosis and treatment act and measure extracted by the characteristic generation unit as an explanatory variable and the index value calculated by the index calculation unit as an objective variable.

(9) An analysis method for analyzing an effect of a diagnosis and treatment act and measure by a computer,
the computer including an arithmetic device that executes a predetermined processing and a storage device connected to the arithmetic device,
the analysis method including:

the arithmetic device executing an input step of receiving an analysis condition;

the arithmetic device executing an event detection step of extracting a disease developing event; and

the arithmetic device executing a cost calculation step of calculating costs of a diagnosis and treatment act that relate to a disease name to be analyzed and are generated after a period of the disease developing event extracted in the event detection step.

(10) The analysis method according to aspect (9), wherein in the cost calculation step, the arithmetic device calculates costs of a diagnosis and treatment act that relate to a disease name the same as the disease name to be analyzed and are generated after the period of the disease developing event.

(11) The analysis method according to aspect (9), wherein in the cost calculation step,
the arithmetic device specifies a diagnosis and treatment act for a disease name related to the disease name to be analyzed by referring to related disease name information in which a related disease name is stored, and
the arithmetic device calculates costs of a diagnosis and treatment act of the disease name to be analyzed and the specified diagnosis and treatment act that are generated after the period of the disease developing event.

(12) The analysis method according to aspect (9), further including:
the arithmetic device executing an economic evaluation calculation step of totalizing medical costs of the disease name to be analyzed by dividing patients into an execution group of a specified diagnosis and treatment act and a non-execution group of the specified diagnosis and treatment act.

(13) The analysis method according to aspect (12), wherein,

in the economic evaluation calculation step, the arithmetic device totalizes medical costs of the disease name to be analyzed for each of a plurality of diagnosis and treatment acts by dividing patients into an execution group of a corresponding diagnosis and treatment act and a non-execution group of the diagnosis and treatment act.

(14) The analysis method according to aspect (13), further including:

the arithmetic device executing a screen configuration processing step of generating screen display data to be output to a display device, wherein

in the economic evaluation calculation step, the arithmetic device totalizes medical costs of all disease names to be analyzed or a selected disease name to be analyzed for each of a plurality of diagnosis and treatment acts by dividing patients into an execution group of a corresponding diagnosis and treatment act and a non-execution group of the diagnosis and treatment act, and

in the screen configuration processing step, the arithmetic device generates display data for displaying economic evaluations of the plurality of diagnosis and treatment acts in a descending order of a difference of totalized medical costs between the execution group and the non-execution group.

(15) The analysis method according to aspect (9), further including:

the arithmetic device executing an economic evaluation calculation step of totalizing medical costs of the disease name to be analyzed by dividing patients into a group in which a specified diagnosis and treatment act is executed within a predetermined period from the disease developing event and a group in which the specified diagnosis and treatment act is executed after the predetermined period.

(21) An analysis system that analyzes an effect of a diagnosis and treatment act and measure and is implemented by a computer including an arithmetic device that executes a predetermined processing and a storage device connected to the arithmetic device, the analysis system including:

an input unit that receives a period to be analyzed and a disease to be analyzed;

an event detection unit that extracts a disease developing event;

a relationship extraction unit that calculates a time series relationship between a period of the disease developing event extracted by the event detection unit and an execution period of the diagnosis and treatment act and measure;

a characteristic generation unit that generates a characteristic amount of the diagnosis and treatment act and measure in consideration of the time series relationship based on the time series relationship calculated by the relationship extraction unit and an execution amount of the diagnosis and treatment act and measure;

an index calculation unit that calculates an index value indicating medical care quality based on clinical data including a history of the diagnosis and treatment act and measure and an examination result of a patient; and

an effect extraction unit that extracts a diagnosis and treatment act and measure having a good index value by setting the characteristic amount of the diagnosis and treatment act and measure extracted by the characteristic generation unit as an explanatory variable and the index value calculated by the index calculation unit as an objective variable.

(22) The analysis system according to aspect (21), wherein

the characteristic generation unit generates the characteristic amount such that a large value is given to a diagnosis and treatment act and measure executed at a timing close to the period of the disease developing event.

(23) The analysis system according to aspect (22), wherein

the characteristic generation unit generates the characteristic amount using the following formula in which $R_{ij}(t)$ represents the time series relationship calculated by the relationship extraction unit.

In the following formula, i represents a patient, j represents a diagnosis and treatment act and measure, t represents time, and f(t) is a monotone decreasing function that decreases with elapse of time.

[Formula 3]

$$Xij = \sum_t f(t)Rij(t)$$

$$Rij(t) = \begin{cases} 1 \text{ if } Aj \text{ was executed at the related month } t(= Execution\_Month(Aj) - M(i)) \\ 0 \text{ otherwise} \end{cases}$$

$$f(t) = \lfloor t \text{ divide } A \rfloor, \qquad (A \text{ is constant.})$$

(24) The analysis system according to aspect (21), wherein
the characteristic generation unit generates the characteristic amount such that a large value is given to a continuously executed diagnosis and treatment act and measure.
(25) The analysis system according to aspect (24), wherein
the characteristic generation unit generates a characteristic amount using the following formula in which Rij (t) represents the time series relationship calculated by the relationship extraction unit.
In the following formula, i represents a patient, j represents a diagnosis and treatment act and measure, t represents time, and f(t) is a monotone decreasing function that decreases with elapse of time.

[Formula 4]

$$Xij = \frac{(\sum_t f(t)Rij(t))^2}{\sum_t (f(t)Rij(t))^2}$$

$$Rij(t) = \begin{cases} 1 \text{ if } Aj \text{ was executed at the related month } t(= Execution\_Month(Aj) - M(i)) \\ 0 \text{ otherwise} \end{cases}$$

$$f(t) = \lfloor t \text{ divide } A \rfloor, \qquad (A \text{ is constant.})$$

(26) The analysis system according to aspect (21), further including:

a knowledge generation unit that extracts a diagnosis and treatment act and measure related to a disease from a history of the diagnosis and treatment act and measure and the clinical data and records the extracted diagnosis and treatment act and measure in knowledge data, wherein
the storage device records the knowledge data including a relationship between the diagnosis and treatment act and measure and the disease, and
the event detection unit extracts a period of the disease developing event by referring to the knowledge data.

(27) The analysis system according to aspect (21), wherein
the effect extraction unit extracts a diagnosis and treatment act and measure having a good index value by setting the characteristic amount of the diagnosis and treatment act extracted by the characteristic generation unit and an index value extracted by the index calculation unit during an initial period of the period to be analyzed as explanatory variables and setting an index value extracted by the index calculation unit during a final period of the period to be analyzed as an objective variable.

(28) An analysis method for analyzing an effect of a diagnosis and treatment act and measure by a computer,
the computer including an arithmetic device that executes a predetermined processing and a storage device connected to the arithmetic device,
the analysis method including:

the arithmetic device executing an input step of receiving a period to be analyzed and a disease to be analyzed;
the arithmetic device executing an event detection step of extracting a disease developing event;
the arithmetic device executing a relationship extraction step of calculating a time series relationship between a period of the disease developing event extracted in the event detection step and an execution period of the diagnosis and treatment act and measure;
the arithmetic device executing a characteristic generation step of generating a characteristic amount of the diagnosis and treatment act and measure in consideration of the time series relationship based on the time series relationship calculated in the relationship extraction step and an execution amount of the diagnosis and treatment act and measure;
the arithmetic device executing an index calculation step of calculating an index value for evaluating medical care quality based on clinical data including a history of the diagnosis and treatment act and measure and an examination result of a patient; and
the arithmetic device executing an effect extraction step of extracting a diagnosis and treatment act and measure having a good index value by setting the characteristic amount of the diagnosis and treatment act and measure extracted in the characteristic generation step as an explanatory variable and setting the index value calculated in the index calculation step as an objective variable.

(29) The analysis method according to aspect (28), wherein
in the characteristic generation step, the arithmetic device generates the characteristic amount such that a large value is given to a diagnosis and treatment act and measure executed at timing close to the period of the disease developing event in time.

(30) The analysis method according to aspect (29), wherein
in the characteristic generation step, the arithmetic device generates a characteristic amount using the following formula in which Rij (t) represents the time series relationship calculated in the relationship extraction step.
In the following formula, i represents a patient, j represents a diagnosis and treatment act and measure, t represents time, and f(t) is a monotone decreasing function that decreases with elapse of time.

[Formula 5]

$$Xij = \sum_t f(t)Rij(t)$$

$Rij(t)$
$$= \begin{cases} 1 \text{ if } Aj \text{ was executed at the related month } t(= Execution\_Month(Aj) - M(i)) \\ 0 \text{ otherwise} \end{cases}$$

$$f(t) = \lfloor t \ divide \ A \rfloor, \qquad (A \text{ is constant.})$$

(31) The analysis method according to aspect (28), wherein
in the characteristic generation step, the arithmetic device generates the characteristic amount such that a large value is given to a continuously executed diagnosis and treatment act and measure.
(32) The analysis method according to aspect (31), wherein
in the characteristic generation step, the arithmetic device generates a characteristic amount using the following formula in which Rij (t) represents the time series relationship calculated in the relationship extraction step.
In the following formula, i represents a patient, j represents a diagnosis and treatment act and measure, t represents time, and f(t) is a monotone decreasing function that decreases with elapse of time.

[Formula 6]

$$Xij = \frac{(\sum_t f(t)Rij(t))^2}{\sum_t (f(t)Rij(t))^2}$$

$Rij(t)$
$$= \begin{cases} 1 \text{ if } Aj \text{ was executed at the related month } t(= Execution\_Month(Aj) - M(i)) \\ 0 \text{ otherwise} \end{cases}$$

$$f(t) = \lfloor t \ divide \ A \rfloor, \qquad (A \text{ is constant.})$$

(33) The analysis method according to aspect (28), further including:

the arithmetic device executing a knowledge generation step of extracting a diagnosis and treatment act and measure related to a disease from a history of the diagnosis and treatment act and measure and the clinical data and recording the extracted diagnosis and treatment act and measure as knowledge data, wherein the storage device records the knowledge data including a relationship between the diagnosis and treatment act and measure and the disease, and

in the event detection step, the arithmetic device extracts a period of the disease developing event by referring to the knowledge data.

(34) The analysis method according to aspect (28), wherein

in the effect extraction step, the arithmetic device extracts a diagnosis and treatment act and measure having a good index value by setting the characteristic amount of the diagnosis and treatment act extracted in the characteristic generation step and an index value extracted in the index calculation step during an initial period of the period to be analyzed as explanatory variables and setting an index value extracted in the index calculation step during a final period of the period to be analyzed as an objective variable.

The invention is not limited to the embodiment described above, and includes various modifications and equivalent configurations within the scope of the appended claims. For example, the embodiment described above has been described in detail for easy understanding of the invention, and the invention is not necessarily limited to those having all the configurations described above. A part of configurations of one embodiment can be replaced with a configuration of another embodiment. The configuration of one embodiment can also be added to the configuration of another embodiment. A part of the configurations of the embodiment may be added to, deleted from, and replaced with another configuration.

The configurations, functions, processing units, processing methods, and the like described above may be partially or entirely implemented by hardware such as through a design using an integrated circuit, or may be implemented by software by a processor interpreting and executing programs for realizing respective functions.

Information such as a program, a table, and a file for realizing each function can be stored in a storage device such as a memory, a hard disk, and a solid state drive (SSD), or a recording medium such as an IC card, an SD card, and a DVD.

Control lines and information lines indicate those what are considered necessary for description, and not all the control lines and the information lines are necessarily shown in a product. In practice, it may be considered that almost all the configurations are connected to one another.

The invention relates to a technique of a hospital information system in the medical field, and is particularly useful as a technique for supporting an effect analysis of a diagnosis and treatment act.

## Claims

1. An analysis system that analyzes an effect of a medical care act and is implemented by a computer including an arithmetic device that executes a predetermined processing and a storage device connected to the arithmetic device, the analysis system comprising:

    an input unit that receives medical care act data, clinical data including an examination result of a patient, medical cost data, and a medical care act and clinical data of a patient to be analyzed;
    an event detection unit that detects a disease developing event;
    a relationship extraction unit that extracts a correlation between the detected disease developing event and a medical care act based on time series information;
    an evaluation index calculation unit that calculates an index value indicating medical care quality based on the received medical care act data and clinical data;
    a cost calculation unit that calculates, by using the medical cost data, costs of a medical care act that relate to a disease name to be analyzed and are generated after a period of the detected disease developing event; and
    an extraction unit that extracts, by using at least one of the calculated index value and the calculated costs, the received medical care act and clinical data of the patient to be analyzed and the correlation extracted by the relationship extraction unit, a medical care act to be recommended to the patient to be analyzed.

2. The analysis system according to claim 1, wherein the extraction unit includes

    a medical care act extraction unit that extracts, by using the received medical care act and clinical data of the patient to be analyzed and the correlation extracted by the relationship extraction unit, a future candidate medical care act for the patient to be analyzed, and
    a candidate extraction unit that extracts a medical care act to be recommended to the patient from the extracted future candidate medical care act by using at least one of the calculated index value and the calculated costs.

3. The analysis system according to claim 2, wherein the input unit receives a selection of a medical care act serving as a starting point of a relative period from medical care acts executed on a presented patient to be analyzed, and the medical care act extraction unit extracts a future candidate medical care act based on a comparison result between a predetermined threshold and a statistical value of a relative period between the selected medical care act serving as the starting point and the extracted candidate medical care act.

4. The analysis system according to claim 3, wherein the input unit presents a medical care act having a high importance degree among the medical care acts executed on the patient to be analyzed.

5. The analysis system according to claim 1, further comprising:
a screen configuration processing unit that generates screen data for outputting the medical care act of the patient to be analyzed that is received by the input unit and the medical care act extracted by the extraction unit.

6. The analysis system according to claim 3, further comprising:
a screen configuration processing unit that generates screen data for outputting a variance of the relative period.

7. The analysis system according to claim 3, wherein the medical care act extraction unit sets the threshold using the statistical value of the relative period.

8. The analysis system according to claim 3, further comprising:
a screen configuration processing unit that generates screen data in a manner in which medical costs of the extracted medical care act are capable of being compared between a group having a long relative period and a group having a short relative period.

9. An analysis method for analyzing an effect of a medical care act by a computer,
the computer including an arithmetic device that executes a predetermined processing and a storage device connected to the arithmetic device,
the analysis method comprising:

the arithmetic device executing an input step of receiving medical care act data, clinical data including an examination result of a patient, medical cost data, and a medical care act and clinical data of a patient to be analyzed;
the arithmetic device executing an event detection step of detecting a disease developing event;
the arithmetic device executing a relationship extraction step of extracting a correlation between the detected disease developing event and a medical care act based on time series information;
the arithmetic device executing an evaluation index calculation step of calculating an index value indicating medical care quality based on the received medical care act data and clinical data;
the arithmetic device executing a cost calculation step of calculating, by using the medical cost data, costs of a medical care act that relate to a disease name to be analyzed and are generated after a period of the detected disease developing event; and
the arithmetic device executing an extraction step of extracting, by using at least one of the calculated index value and the calculated costs, the received medical care act and clinical data of the patient to be analyzed and the correlation extracted in the relationship extraction step, a medical care act to be recommended to the patient to be analyzed.

10. The analysis method according to claim 9, wherein the extraction step includes

the arithmetic device executing a medical care act extraction step of extracting, by using the received medical care act and clinical data of the patient to be analyzed and the correlation extracted in the relationship extraction step, a future candidate medical care act for the patient to be analyzed, and
the arithmetic device executing a candidate extraction step of extracting a medical care act to be recommended to the patient from the extracted future candidate medical care act by using at least one of the calculated index value and the calculated costs.

11. The analysis method according to claim 10, wherein
in the input step, the arithmetic device receives a selection of a medical care act serving as a starting point of a relative period from medical care acts executed on a presented patient to be analyzed, and
in the medical care act extraction step, the arithmetic device extracts a future candidate medical care act based on a comparison result between a predetermined threshold and a statistical value of a relative period between the selected medical care act serving as the starting point and the extracted candidate medical care act.

12. The analysis method according to claim 11, wherein in the input step, the arithmetic device presents a medical care act having a high importance degree among the medical care acts executed on the patient to be analyzed.

13. The analysis method according to claim 9, further comprising:
the arithmetic device executing a screen configuration processing step of generating screen data for outputting the medical care act of the patient to be analyzed that is received in the input step and the medical care act extracted in the extraction step.

14. The analysis method according to claim 11, wherein in the medical care act extraction step, the arithmetic device sets the threshold by using the statistical value of the relative period.

15. The analysis method according to claim 11, further comprising:
the arithmetic device executing a screen configuration processing step of generating screen data in a manner in which medical costs of the extracted medical care act are capable of being compared between a group having a long relative period and a group having a short relative period.

# FIG. 1

# FIG. 2

101
DIAGNOSIS AND TREATMENT ACT DB

102
CLINICAL DB

100
MEDICAL COST DB

DIAGNOSIS AND TREATMENT ACT AND MEASURE ECONOMIC VALUE EVALUATION SYSTEM

103
EXTERNAL DB COOPERATION UNIT

110
TARGET DISEASE TOTAL COST CALCULATION UNIT

104
DISEASE DEVELOPING EVENT DETECTION UNIT

106
DISEASE DEVELOPING AND DIAGNOSIS AND TREATMENT ACT RELATIONSHIP EXTRACTION UNIT

108
EVALUATION INDEX CALCULATION UNIT

105
DISEASE DEVELOPING KNOWLEDGE DB

107
DISEASE DEVELOPING TIME SERIES INFORMATION CONVOLUTION UNIT

109
DIAGNOSIS AND TREATMENT EFFECT EXTRACTION UNIT

111
MEDICAL CARE ECONOMIC EVALUATION CALCULATION UNIT

112
TARGET PATIENT MEDICAL CARE ACT EXTRACTION UNIT

113
CANDIDATE EXTRACTION UNIT

114
SCREEN CONFIGURATION PROCESSING UNIT

115
INPUT UNIT

116
DISPLAY UNIT

## FIG. 3

DIAGNOSIS AND TREATMENT ACT AND MEASURE ECONOMIC VALUE EVALUATION SYSTEM

200

| INPUT DEVICE |
|---|

202

| MEMORY |
|---|

| OUTPUT DEVICE |
|---|

201

| CENTRAL PROCESSING UNIT |
|---|

203

| AUXILIARY STORAGE DEVICE |
|---|

204

## FIG. 4

START

INPUT DISEASE TO BE ANALYZED AND PERIOD TO BE ANALYZED VIA INPUT UNIT — S301

REFER TO DISEASE DEVELOPING KNOWLEDGE DB TO EXTRACT DIAGNOSIS AND TREATMENT ACT INFORMATION OR EXAMINATION INFORMATION (DISEASE DEVELOPING EVENT) CORRESPONDING TO INPUT DISEASE NAME DURING INPUT PERIOD FROM DIAGNOSIS AND TREATMENT ACT DB AND CLINICAL DB — S302

CALCULATE TIME SERIES RELATIONSHIP BETWEEN PERIODS OF EXTRACTED DISEASE DEVELOPING EVENT FOR EACH DIAGNOSIS AND TREATMENT ACT OR MEASURE STORED IN DIAGNOSIS AND TREATMENT ACT DATABASE — S303

GENERATE CHARACTERISTIC AMOUNT IN CONSIDERATION OF CALCULATED TIME SERIES INFORMATION AND EXECUTION AMOUNT OF EACH DIAGNOSIS AND TREATMENT ACT FOR EACH DIAGNOSIS AND TREATMENT ACT OR MEASURE — S304

CALCULATE INDEX VALUE FOR EVALUATING MEDICAL CARE QUALITY FROM DIAGNOSIS AND TREATMENT ACT DB AND CLINICAL DB — S305

SET GENERATED CHARACTERISTIC AMOUNT OF DIAGNOSIS AND TREATMENT ACT OR MEASURE AND INITIAL VALUE OF CALCULATED INDEX VALUE AS EXPLANATORY VARIABLES, SET CALCULATED INDEX VALUE AS OBJECTIVE VARIABLE, AND EXTRACT HIGHLY EFFECTIVE DIAGNOSIS AND TREATMENT ACT — S306

CALCULATE ALL MEDICAL COSTS AFTER DISEASE DEVELOPING BY ALSO USING PERIOD OF EXTRACTED DISEASE DEVELOPING EVENT — S307

CALCULATE ECONOMIC EVALUATION FOR DIAGNOSIS AND TREATMENT ACT EXTRACTED IN S306 — S308

EXTRACT FUTURE CANDIDATE MEDICAL CARE ACT AND MEASURE FOR PATIENT TO BE ANALYZED FROM MEDICAL CARE ACT AND MEASURE AND CLINICAL DATA OF PATIENT TO BE ANALYZED, AND ECONOMIC EVALUATION RESULT CALCULATED IN S308 — S309

END

## FIG. 5

109
DIAGNOSIS AND
TREATMENT EFFECT
EXTRACTION UNIT

107
DISEASE DEVELOPING TIME
SERIES INFORMATION
CONVOLUTION UNIT

108
EVALUATION INDEX
CALCULATION UNIT

S302, S303, S304
REQUEST CHARACTERISTIC
AMOUNT OF DIAGNOSIS AND
TREATMENT ACT

CHARACTERISTIC AMOUNT OF
DIAGNOSIS AND TREATMENT ACT

S305

REQUEST MEDICAL CARE QUALITY
(EVALUATION INDEX VALUE)

MEDICAL CARE QUALITY
(EVALUATION INDEX VALUE)

## FIG. 6

| CONDITION SETTING AND PROCESSING RESULT DISPLAY | ▬ ⬜ ✕ |

DISEASE NAME ▼ DIABETES

QI INDEX ▼ BLOOD GLUCOSE CONTROL

TARGET PERIOD ▼ 2013 ▼ 2016

EFFECTIVENESS
ANALYSIS
5011

EVALUATION INDEX
CALCULATION
5012

501

502

# FIG. 7

S302

```
                    START
                      │
                      ▼
┌─────────────────────────────────────────────┐  S3021
│  ACQUIRE DIAGNOSIS AND TREATMENT ACT DATA OF │
│        TARGET PATIENT AND CLINICAL DATA      │
│          VIA EXTERNAL DB COOPERATION UNIT    │
└─────────────────────────────────────────────┘
                      │
                      ▼
┌─────────────────────────────────────────────┐  S3022
│ EXTRACT CANDIDATE DISEASE DEVELOPING DATE FROM CLINICAL │
│    DATA BASED ON DEFINITION OF DISEASE DEVELOPING       │
└─────────────────────────────────────────────┘
                      │
                      ▼
┌─────────────────────────────────────────────┐  S3023
│    EXTRACT ABSOLUTE DATE AND TIME AND PATIENT CODE      │
│         FOR DIAGNOSIS AND TREATMENT ACT THAT            │
│      MATCHES DISEASE DEVELOPING KNOWLEDGE DATA          │
└─────────────────────────────────────────────┘
                      │
                      ▼
┌─────────────────────────────────────────────┐  S3024
│  DETERMINE EARLIEST DATE AS DISEASE DEVELOPING PERIOD   │
│    FOR EACH PATIENT BASED ON EXTRACTION RESULTS         │
│              OF S3022 AND S3023                         │
└─────────────────────────────────────────────┘
                      │
                      ▼
                    END
```

# FIG. 8

| PATIENT CODE | GENDER | AGE | DISEASE NAME | DIAGNOSIS AND TREATMENT ACT | EXECUTION DATE |
|---|---|---|---|---|---|
| P0 | MALE | 60 | DIABETES | DPP4 | 13/5/1 |
| P0 | MALE | 60 | DIABETES | SGLT2 | 13/6/1 |
| P1 | FEMALE | 70 | DIABETES | HbA1c EXAMINATION | 14/5/1 |
| P1 | FEMALE | 70 | DIABETES | BODY TEMPERATURE MEASUREMENT | 14/6/1 |
| ⋮ | ⋮ | ⋮ | ⋮ | ⋮ | ⋮ |

DIAGNOSIS AND TREATMENT ACT DATA

## FIG. 9

| PATIENT CODE | EXECUTION DATE | HbA1c | LDL |
|---|---|---|---|
| P0 | 13/4/1 | 6.0 | 130 |
| P0 | 14/7/1 | 7.0 | 150 |
| P1 | 13/5/1 | 6.8 | 140 |
| ⋮ | ⋮ | ⋮ | ⋮ |

CLINICAL DATA

## FIG. 10

| DISEASE NAME | DIAGNOSIS AND TREATMENT ACT |
|---|---|
| DIABETES | DPP4 |
| DIABETES | SGLT2 |
| DIABETES | HbA1c EXAMINATION |
| ISCHEMIC HEART DISEASE | CATHETER EXAMINATION |
| ⋮ | ⋮ |

DISEASE DEVELOPING KNOWLEDGE DATA

## FIG. 11

| PATIENT CODE | DISEASE NAME | DISEASE DEVELOPING DATE |
|:---:|:---:|:---:|
| P0 | DIABETES | 13/5/1 |
| P1 | DIABETES | 13/5/1 |
| ⋮ | ⋮ | ⋮ |

DISEASE DEVELOPING DATE MANAGEMENT TABLE

## FIG. 12

S303

START

S3031
ACQUIRE DISEASE DEVELOPING DATE OF EACH PATIENT FROM DISEASE DEVELOPING EVENT DETECTION UNIT

S3032
ACQUIRE DIAGNOSIS AND TREATMENT ACT OF TARGET PATIENT AND ABSOLUTE DATES OF DIAGNOSIS AND TREATMENT ACT VIA EXTERNAL DB COOPERATION UNIT

S3033
CALCULATE RELATIVE DAYS FROM DISEASE DEVELOPING DATE FOR EACH DIAGNOSIS AND TREATMENT ACT OR MEASURE OF EACH PATIENT

END

## FIG. 13

| 106 | 104 | 103 |
|---|---|---|
| DISEASE DEVELOPING AND DIAGNOSIS AND TREATMENT ACT RELATIONSHIP EXTRACTION UNIT | DISEASE DEVELOPING EVENT DETECTION UNIT | EXTERNAL DB COOPERATION UNIT |

S3031

REQUEST DISEASE DEVELOPING DATE OF EACH PATIENT

DISEASE DEVELOPING DATE OF EACH PATIENT

S3032

REQUEST DIAGNOSIS AND TREATMENT ACT OF TARGET PATIENT AND EXECUTION DATE OF DIAGNOSIS AND TREATMENT ACT

DIAGNOSIS AND TREATMENT ACT OF TARGET PATIENT AND EXECUTION DATE OF DIAGNOSIS AND TREATMENT ACT

## FIG. 14

| PATIENT CODE | GENDER | AGE | DIAGNOSIS AND TREATMENT PROCESS | DISEASE DEVELOPING DATE | EXECUTION DATE | RELATIVE DAYS |
|---|---|---|---|---|---|---|
| P0 | MALE | 60 | DPP4 | 13/5/1 | 13/5/1 | 0 |
| P0 | MALE | 60 | SGLT2 | 13/5/1 | 13/6/1 | 31 |
| P1 | FEMALE | 70 | HbA1c EXAMINATION | 13/5/1 | 14/5/1 | 365 |
| ... | ... | ... | ... | | ... | ... |

DIAGNOSIS AND TREATMENT ACT TIME SERIES INFORMATION TABLE

# FIG. 15

| PATIENT CODE | DPP4 | SGLT2 | HbA1c EXAMINATION | · · · |
|---|---|---|---|---|
| P0 | 2 | 1 | 0 | · · · |
| P1 | 0 | 0 | 1 | · · · |
| · · · | · · · | · · · | · · · | · · · |

TIME (EARLY DIAGNOSIS)
CONTINUITY
THE NUMBER OF TIMES

| PATIENT CODE | DPP4 | SGLT2 | HbA1c EXAMINATION | · · · |
|---|---|---|---|---|
| P0 | 10 | 5 | 0 | · · · |
| P1 | 0 | 0 | 1 | · · · |
| · · · | · · · | · · · | · · · | · · · |

DIAGNOSIS AND TREATMENT ACT CHARACTERISTIC AMOUNT TABLE

## FIG. 16

S306

START

ACQUIRE CHARACTERISTIC AMOUNT OF DIAGNOSIS AND TREATMENT ACT FROM DISEASE DEVELOPING TIME SERIES INFORMATION CONVOLUTION UNIT — S3061

ACQUIRE INITIAL VALUE OF EFFECT CHARACTERISTIC AMOUNT (INDEX VALUE) VIA EVALUATION INDEX CALCULATION UNIT — S3062

INTEGRATE RESULTS OF S3061 AND S3062 TO CREATE CHARACTERISTIC AMOUNT VECTOR FOR EACH PATIENT — S3063

ACQUIRE FINAL RESULT OF EFFECT CHARACTERISTIC AMOUNT (INDEX VALUE) VIA EVALUATION INDEX CALCULATION UNIT — S3064

SELECT CHARACTERISTIC THAT AFFECTS FINAL RESULT OF EFFECT CHARACTERISTIC AMOUNT FROM CHARACTERISTIC AMOUNT VECTOR GENERATED IN S3063 — S3065

END

# FIG. 17

S307

START

ACQUIRE DISEASE DEVELOPING DATE OF EACH PATIENT FROM DISEASE DEVELOPING EVENT DETECTION UNIT — S3071

ACQUIRE MEDICAL COSTS OF TARGET PATIENT AFTER DISEASE DEVELOPING DATE VIA EXTERNAL DB COOPERATION UNIT — S3072

CALCULATE TOTAL MEDICAL COSTS FROM DISEASE DEVELOPING DATE FOR EACH PATIENT — S3073

END

# FIG. 18

| MAIN DISEASE NAME | RELATED DISEASE NAME |
|---|---|
| DIABETES | HYPERTENSION |
| DIABETES | DYSLIPIDEMIA |
| DIABETES | NEPHROPATHY |
| MYOCARDIAL INFARCTION | PNEUMONIA |
| ⋮ | ⋮ |

RELATED DISEASE NAME TABLE

## FIG. 19

| PATIENT CODE | DISEASE NAME | EXECUTION DATE | MEDICAL COST |
|---|---|---|---|
| P0 | DIABETES | 13/5/1 | 2000 |
| P0 | DIABETES | 13/6/1 | 3000 |
| P1 | HYPERLIPIDEMIA | 12/6/1 | 1000 |
| P1 | HYPERLIPIDEMIA | 13/6/1 | 1000 |
| P1 | DIABETES | 14/5/1 | 2000 |
| P1 | DIABETES | 14/6/1 | 3000 |
| ⋮ | ⋮ | ⋮ | |

MEDICAL COST TABLE

## FIG. 20

S308

START

DIVIDE TARGET PATIENTS INTO TWO GROUPS BASED ON WHETHER SELECTED DIAGNOSIS AND TREATMENT ACT IS EXECUTED — S3081

CALCULATE AVERAGE VALUE OF TOTAL MEDICAL COSTS FOR EACH GROUP — S3082

END

# FIG. 21

| CONDITION SETTING AND PROCESSING RESULT DISPLAY | ▬ ⬒ ✕ |
|---|---|

DISEASE NAME ▼ DIABETES

QI INDEX ▼ BLOOD GLUCOSE CONTROL

TARGET PERIOD ▼ 2013 ▼ 2016

EFFECTIVENESS ANALYSIS — 5011

EVALUATION INDEX CALCULATION — 5012

501

502

| CHARACTERISTIC | SELECT | IMPORTANCE DEGREE |
|---|---|---|
| SGLT2 INHIBITOR | ■ | 1.0 |
| HbA1c EXAMINATION | ☐ | 0.8 |
| DPP4 INHIBITOR | ☐ | 0.6 |

# FIG. 22

CONDITION SETTING AND PROCESSING RESULT DISPLAY  ▭ ▭ ✕

DISEASE NAME  ▼ DIABETES

QI INDEX  ▼ BLOOD GLUCOSE CONTROL

TARGET PERIOD  ▼ 2013  ▼ 2016

EFFECTIVENESS ANALYSIS — 5011

EVALUATION INDEX CALCULATION — 5012

501

502

SGLT2 (EXECUTION)

SGLT2 (NON-EXECUTION)

# FIG. 23

S309

START

RECEIVE PROCESS HAVING HIGH IMPORTANCE DEGREE
CALCULATED IN S306 AMONG MEDICAL CARE PROCESSES
FOR PATIENTS TO BE ANALYZED

S3091

AMONG DIAGNOSIS AND TREATMENT PROCESSES EXTRACTED IN S303,
CALCULATE RELATIVE DAYS STARTING FROM
WHEN PROCESS IS RECEIVED IN S3091 FOR EACH PATIENT

S3092

CALCULATE AVERAGE VALUE OF RELATIVE DAYS EXTRACTED IN S3092
FOR EACH DIAGNOSIS AND TREATMENT PROCESS

S3093

EXTRACT DIAGNOSIS AND TREATMENT PROCESS
WHOSE AVERAGE VALUE OF RELATIVE DAYS CALCULATED IN S3093
IS EQUAL TO OR LARGER THAN PRESET THRESHOLD

S3094

EXTRACT PAIRS OF ECONOMIC EVALUATION GENERATED IN S308 AND
DIAGNOSIS AND TREATMENT ACT USING DIAGNOSIS AND
TREATMENT PROCESS EXTRACTED IN S3094 AS KEY

S3095

EXTRACT PAIR IN WHICH IMPORTANCE DEGREE AND/OR ECONOMIC
EVALUATION IS IN UPPER LEVEL AMONG PAIRS EXTRACTED IN S3095

S3096

END

## FIG. 24

| CONDITION SETTING AND PROCESSING RESULT DISPLAY | — ⬚ ✕ |

DISEASE NAME ▼ DIABETES

TARGET PERIOD ▼ 2013 ▼ 2016

PATIENT CODE ▼ P100

EXECUTED DIAGNOSIS AND TREATMENT ACT — 27011

RECOMMENDED DIAGNOSIS AND TREATMENT ACT — 27012

2701

2702

| START POINT | EXECUTED DIAGNOSIS AND TREATMENT ACT | EXECUTION DATE | EXECUTION AMOUNT |
|---|---|---|---|
| | HbA1c EXAMINATION | 19/6/1 | 1 |
| | DPP4 INHIBITOR | 19/7/1 | 10 |
| ✔ | HbA1c EXAMINATION | 19/8/1 | 1 |
| | BODY TEMPERATURE MEASUREMENT | 19/8/2 | 1 |

# FIG. 25

| CONDITION SETTING AND PROCESSING RESULT DISPLAY | — ⬓ ✕ |
|---|---|

DISEASE NAME ▼ DIABETES

TARGET PERIOD ▼ 2013 ▼ 2016

PATIENT CODE ▼ P100

| EXECUTED DIAGNOSIS AND TREATMENT ACT |
|---|

27011

| RECOMMENDED DIAGNOSIS AND TREATMENT ACT |
|---|

27012

2701

2703

| RECOMMENDED DIAGNOSIS AND TREATMENT ACT | IMPORTANCE DEGREE | ECONOMIC EVALUATION |
|---|---|---|
| SGLT2 INHIBITOR | 1.0 | 200000 |
| HbA1c EXAMINATION | 0.8 | 100000 |

## FIG. 26

S308

```
            ┌──────────────────┐
            │      START       │
            └──────────────────┘
                     │
                     ▼
┌─────────────────────────────────────────────────┐  S3083
│                                                   │
│                    i = 0                          │
│                                                   │
└─────────────────────────────────────────────────┘
                     │
                     ▼
┌─────────────────────────────────────────────────┐  S3084
│  DIVIDE TARGET PATIENTS INTO TWO GROUPS BASED ON  │
│  WHETHER DIAGNOSIS AND TREATMENT ACT i IS EXECUTED│
└─────────────────────────────────────────────────┘
                     │
                     ▼
┌─────────────────────────────────────────────────┐  S3085
│    CALCULATE AVERAGE VALUE OF TOTAL MEDICAL COSTS │
│                 FOR EACH GROUP                    │
└─────────────────────────────────────────────────┘
                     │
                     ▼
┌─────────────────────────────────────────────────┐  S3086
│              IF i < N, THEN i++                   │
│               ELSE GO TO END                      │
└─────────────────────────────────────────────────┘
                     │
                     ▼
            ┌──────────────────┐
            │       END        │
            └──────────────────┘
```

## FIG. 27

| CONDITION SETTING AND PROCESSING RESULT DISPLAY | ▬ ⬜ ✕ |

DISEASE NAME  ▼ DIABETES

QI INDEX  ▼ BLOOD GLUCOSE CONTROL

TARGET PERIOD  ▼ 2013  ▼ 2016

| EFFECTIVENESS ANALYSIS | — 5011 |

| EVALUATION INDEX CALCULATION | — 5012 |

501

502

| CHARACTERISTIC | IMPORTANCE DEGREE | ECONOMIC EVALUATION |
|---|---|---|
| SGLT2 INHIBITOR | 1.0 | 200000 |
| HbA1c EXAMINATION | 0.8 | 100000 |
| DPP4 INHIBITOR | 0.6 | 20000 |

## FIG. 28

| CONDITION SETTING AND PROCESSING RESULT DISPLAY | — | ⬓ | ✕ |
|---|---|---|---|

QI INDEX ▼ IN-HOSPITAL DAYS

TARGET PERIOD ▼ 2013 ▼ 2016

EFFECTIVENESS ANALYSIS — 5011

EVALUATION INDEX CALCULATION — 5012

501

502

| DISEASE NAME | CHARACTERISTIC | IMPORTANCE DEGREE | ECONOMIC EVALUATION |
|---|---|---|---|
| DIABETES | SGLT2 INHIBITOR | 1.0 | 200000 |
| MYOCARDIAL INFARCTION | CATHETER EXAMINATION | 0.9 | 150000 |
| DIABETES | HbA1c EXAMINATION | 0.8 | 100000 |
| DIABETES | DPP4 INHIBITOR | 0.6 | 20000 |

## FIG. 29

S308

START

DIVIDE TARGET PATIENTS INTO TWO GROUPS BASED ON WHETHER SELECTED DIAGNOSIS AND TREATMENT ACT IS EXECUTED IN EARLY STAGE — S3087

CALCULATE AVERAGE VALUE OF TOTAL MEDICAL COSTS FOR EACH GROUP — S3088

END

# FIG. 30

| CONDITION SETTING AND PROCESSING RESULT DISPLAY | ▬ ⬜ ✕ |

DISEASE NAME ▼ DIABETES

QI INDEX ▼ BLOOD GLUCOSE CONTROL

TARGET PERIOD ▼ 2013 ▼ 2016

EFFECTIVENESS ANALYSIS — 5011

EVALUATION INDEX CALCULATION — 5012

501

502

| CHARACTERISTIC | SELECT | IMPORTANCE DEGREE |
|---|---|---|
| SGLT2 INHIBITOR | ■ | 1.0 |
| HbA1c EXAMINATION | ☐ | 0.8 |
| DPP4 INHIBITOR | ☐ | 0.6 |

■ECONOMIC EVALUATION DURING DIAGNOSIS AND TREATMENT STARTING PERIOD — 503

EARLY STAGE : ▼ 12 MONTH

## FIG. 31

CONDITION SETTING AND PROCESSING RESULT DISPLAY ▬ ⬒ ✕

DISEASE NAME ▼ DIABETES

QI INDEX ▼ BLOOD GLUCOSE CONTROL

TARGET PERIOD ▼ 2013 ▼ 2016

EFFECTIVENESS ANALYSIS — 5011

EVALUATION INDEX CALCULATION — 5012

501

502

SGLT2 (EARLY EXECUTION)    SGLT2 (LATE EXECUTION)

■ ECONOMIC EVALUATION DURING DIAGNOSIS AND TREATMENT STARTING PERIOD — 503

EARLY STAGE: ▼ 12 MONTH

# FIG. 32

# FIG. 33

S309

START

RECEIVE PROCESS HAVING HIGH IMPORTANCE DEGREE
CALCULATED IN S306 AMONG MEDICAL CARE PROCESSES
FOR PATIENTS TO BE ANALYZED — S3091

FOR DIAGNOSIS AND TREATMENT PROCESSES EXTRACTED IN S303,
CALCULATE RELATIVE DAYS STARTING FROM WHEN PROCESS
IS RECEIVED IN S3091 FOR EACH PATIENT — S3092

CALCULATE AVERAGE VALUE OF RELATIVE DAYS EXTRACTED IN S3092
FOR EACH DIAGNOSIS AND TREATMENT PROCESS — S3093

EXTRACT DIAGNOSIS AND TREATMENT PROCESS
WHOSE AVERAGE VALUE OF RELATIVE DAYS CALCULATED IN S3093
IS EQUAL TO OR LARGER THAN PRESET THRESHOLD — S3094

EXTRACT PROCESS HAVING HIGH DELAY RISK
BASED ON RELATIVE DAYS OF EACH PATIENT CALCULATED IN S3092
FOR EACH DIAGNOSIS AND TREATMENT PROCESS EXTRACTED IN S3094 — S3097

EXTRACT PAIRS OF ECONOMIC EVALUATION GENERATED IN S308
AND DIAGNOSIS AND TREATMENT ACT USING DIAGNOSIS
AND TREATMENT PROCESS EXTRACTED IN S3094 AS KEY — S3095

EXTRACT PAIR IN WHICH IMPORTANCE AND/OR ECONOMIC
EVALUATION IS IN UPPER LEVEL AMONG PAIRS EXTRACTED IN S3095
TOGETHER WITH DELAY RISK EXTRACTED IN S3097 — S30961

END

# FIG. 34

| CONDITION SETTING AND PROCESSING RESULT DISPLAY | — ◰ ✕ |
|---|---|

DISEASE NAME ▼ DIABETES

TARGET PERIOD ▼ 2013 ▼ 2016

PATIENT CODE ▼ P100

| EXECUTED DIAGNOSIS AND TREATMENT ACT | 27011 |
|---|---|
| RECOMMENDED DIAGNOSIS AND TREATMENT ACT | 27012 / 2701 |

2702

| RECOMMENDED DIAGNOSIS AND TREATMENT ACT | IMPORTANCE DEGREE | ECONOMIC EVALUATION | DELAY RISK |
|---|---|---|---|
| SGLT2 INHIBITOR | 1.0 | 200000 | |
| HbA1c EXAMINATION | 0.8 | 100000 | ✔ |

# EUROPEAN SEARCH REPORT

**Application Number**

EP 20 18 0447

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/024784 A1 (KIDO KUNIHIKO [JP] ET AL) 26 January 2017 (2017-01-26) * fig. 9, 12, 13, 14; 8, 35-43, 45 et seq., 89 et seq., 106- 123 * ----- | 1-15 | INV. G16H40/20 G06Q50/00 G16H10/60 |

TECHNICAL FIELDS SEARCHED (IPC)

G16H
G06Q

The present search report has been drawn up for all claims

| Place of search | Date of completion of the search | Examiner |
|---|---|---|
| Munich | 13 November 2020 | Wittke, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 18 0447

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2020

| Patent document cited in search report | Publication date | Patent family member(s) | Publication date |
|---|---|---|---|
| US 2017024784 A1 | 26-01-2017 | JP 6189973 B2 | 30-08-2017 |
| | | JP WO2015079552 A1 | 16-03-2017 |
| | | US 2017024784 A1 | 26-01-2017 |
| | | WO 2015079552 A1 | 04-06-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014215761 A

- WO 201577582 A

**Non-patent literature cited in the description**

- **B. LEE et al.** Identification of Type 2 Diabetes Risk Factors Using Phenotypes Consisting of Anthropometry and Triglycerides Based on Machine Learning. *IEEE J. of Biomedical and Health Informatics,* January 2016, vol. 20 (1